# EUROPÄISCHE PATENTSCHRIFT

(11) **EP 2 225 236 B1**
(45) Veröffentlichungstag und Bekanntmachung des Hinweises auf die Patenterteilung: **27.01.2016**
(21) Anmeldenummer: 08851154.8
(22) Anmeldetag: 21.11.2008
(51) Int. Cl.: C07D 401/14, C07D 417/14, C07D 471/04

(54) **ORGANISCHE VERBINDUNGEN**
ORGANIC COMPOUNDS
COMPOSÉS ORGANIQUES

(30) Priorität: 22.11.2007 EP 07121353
(43) Veröffentlichungstag der Anmeldung: 08.09.2010
(73) Patentinhaber: Boehringer Ingelheim International GmbH, 55216 Ingelheim am Rhein (DE)
(72) Erfinder: GOTTSCHLING, Dirk, 55216 Ingelheim am Rhein (DE); DAHMANN, Georg, 55216 Ingelheim am Rhein (DE); DOODS, Henri, 55216 Ingelheim am Rhein (DE); HEIMANN, Annekatrin, 55216 Ingelheim am Rhein (DE); MUELLER, Stephan Georg, 55216 Ingelheim am Rhein (DE); RUDOLF, Klaus, 55216 Ingelheim am Rhein (DE); SCHAENZLE, Gerhard, 55216 Ingelheim am Rhein (DE); STENKAMP, Dirk, 55216 Ingelheim am Rhein (DE)
(74) Vertreter: Simon, Elke Anna Maria
(86) Internationale Anmeldenummer: PCT/EP2008/065963
(87) Internationale Veröffentlichungsnummer: WO 2009/065921

(56) Entgegenhaltungen:
- WO-A-02/22592
- WO-A-2005/030751

## Beschreibung

Gegenstand der vorliegenden Erfindung sind neue CGRP-Antagonisten der allgemeinen Formeln **Ia** und **Ib** 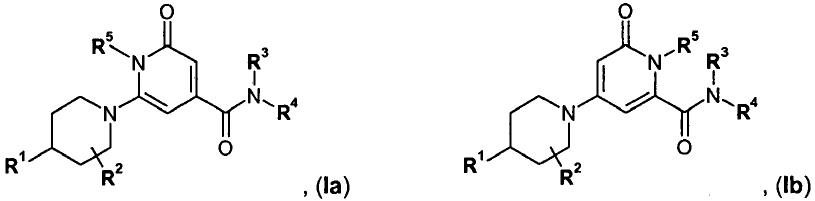 in der **R¹, R², R³, R⁴** und **R⁵** wie nachstehend erwähnt definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

### DETAILLIERTE BESCHREIBUNG DER ERFINDUNG

In der obigen allgemeinen Formeln **Ia** und **Ib** bedeuten in seiner Ausführungsform
- **R¹**: eine Gruppe ausgewählt aus 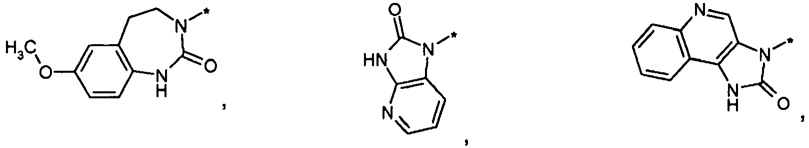 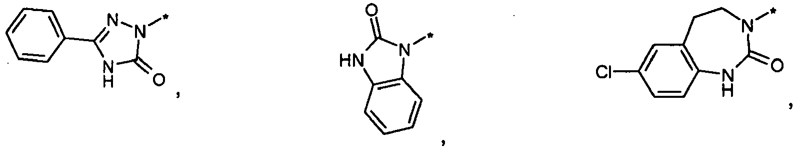 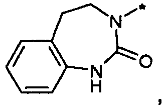
**R²** H,
**R³**
(a) H,
(b) C₁₋₃-Alkyl,
(c) eine C₁₋₃-Alkylgruppe, in der jede Methylengruppe mit bis zu zwei Fluoratomen und jede Methylgruppe mit bis zu drei Fluoratomen substituiert ist, und

**R⁴** H oder eine Gruppe ausgewählt aus 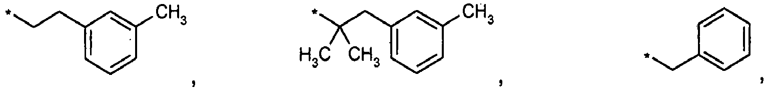 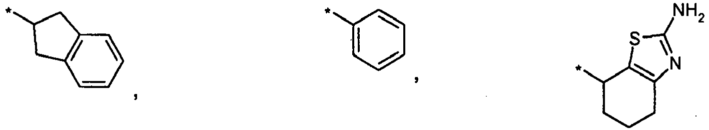 oder
- **R³** und **R⁴**: zusammen mit dem Stickstoffatom, an das sie gebunden sind, eine Gruppe ausgewählt aus 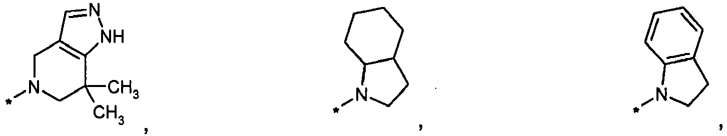 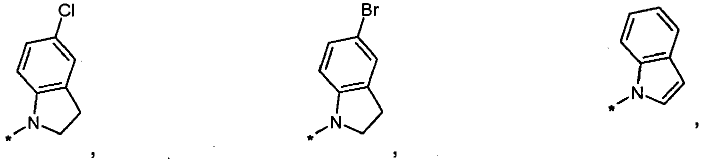 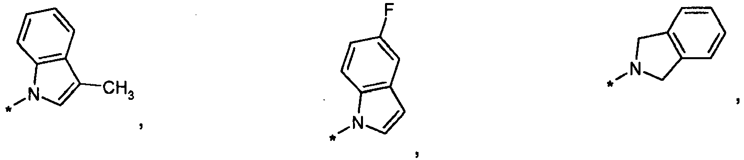 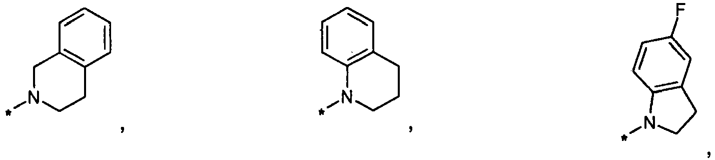 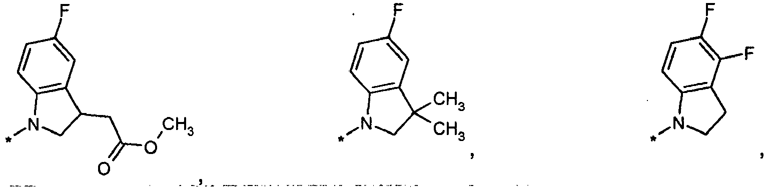 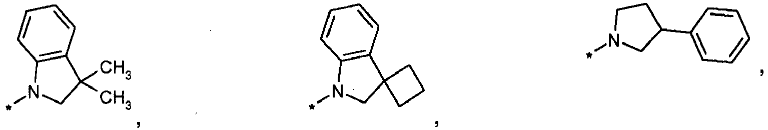 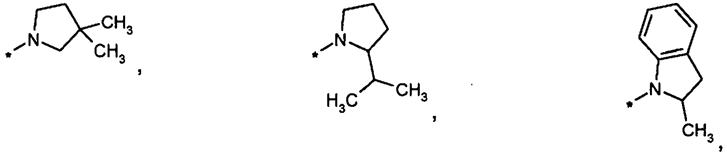 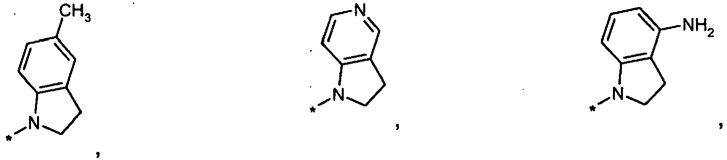 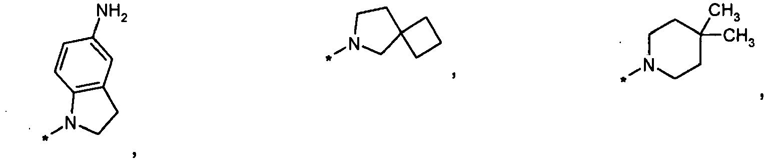 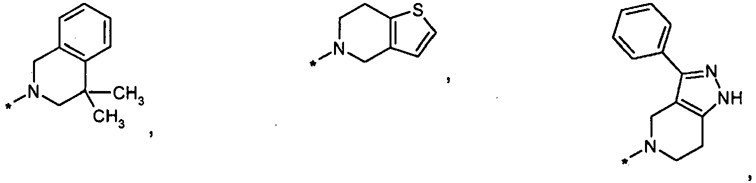 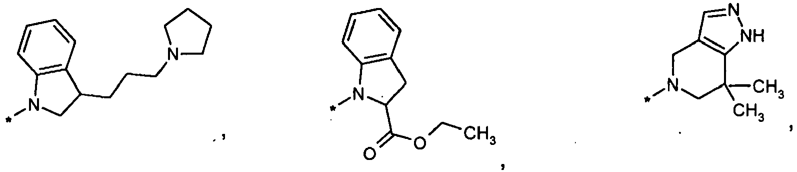 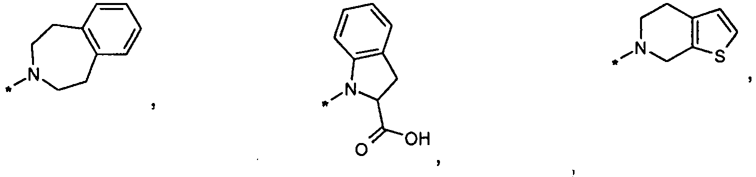 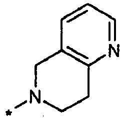 und
- **R⁵**: H oder C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

WO 2005/030751 beschreibt ähnliche 2-oxo-2,3-Dihydroimidazo[4,5-b]pyridin-1-yl Verbindungen und deren Verwendung in der Behandlung von z.B. Krebs.

Als ganz besonders bevorzugte Verbindungen der obigen allgemeinen Formeln **Ia** und **Ib** seien beispielsweise folgende Verbindungen genannt:

| **Nr.** | **Struktur** |
|---|---|
| (1) | 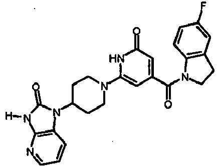 |
| (2) | 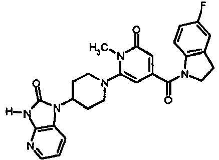 |
| (3) | 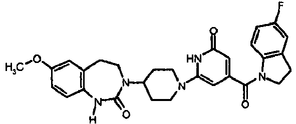 |
| (4) | 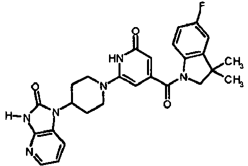 |
| (5) | 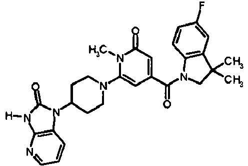 |
| (6) | 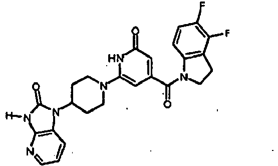 |
| (7) | 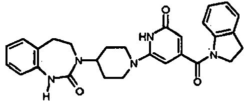 |
| (8) | 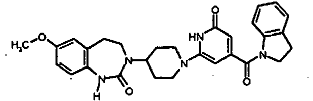 |
| (9) | 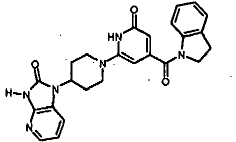 |
| (10) | 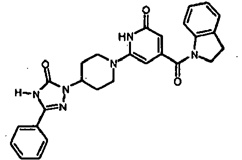 |
| (11) | 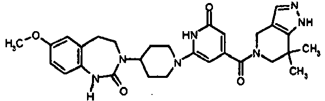 |
| (12) | 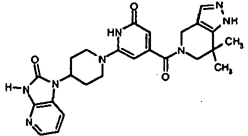 |
| (13) | 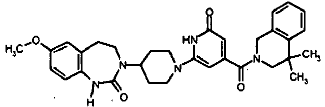 |
| (14) | 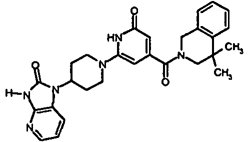 |
| (16) | 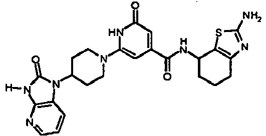 |
| (17) | 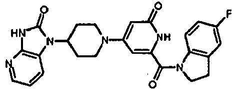 |

deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINITIONEN

Die vorliegende Beschreibung der Erfindung soll in Übereinstimmung mit den Gesetzmäßigkeiten und Regeln der chemischen Bindungen aufgefasst werden.

Die Verbindungen, die von dieser Erfindung umfasst sind, sind jene, die auch chemisch stabil sind.

Soweit nicht anders angegeben, sind alle Substituenten voneinander unabhängig. Sollten an einer Gruppe z. B. mehrere C₁₋₄-Alkylgruppen als Substituenten sein, so könnte, im Fall von drei Substituenten C₁₋₄-Alkyl unabhängig voneinander einmal Methyl, einmal Ethyl und einmal *n*-Propyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden. Beispielsweise wird ein Phenyl-Rest wie folgt dargestellt:

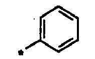

Weiterhin wird das auf den Verknüpfungspunkt folgende Atom des Substituenten als das Atom mit der Positionsnummer 1 verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen, unter dem Begriff "C₁₋₄-Alkyl" werden verzweigte und unverzweigte Alkylgruppen mit 1 bis 4 Kohlenstoffatomen und unter dem Begriff "C₁₋₆-Alkyl" werden verzweigte und unverzweigte Alkylgruppen mit 1 bis 6 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl, *tert*-Butyl, Pentyl, Neopentyl oder *n*-Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Verbindungen der allgemeinen Formeln **Ia** und **Ib** können Säuregruppen besitzen, hauptsächlich Carboxylgruppen, und/oder basische Gruppen wie z.B. Aminofunktionen. Verbindungen der allgemeinen Formel **Ia** und **Ib** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, p-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formeln **Ia** und **Ib** vorhanden ist, sowie die einzelnen optisch aktiven Enantiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen.

Gegenstand der Erfindung ist weiterhin ein Verfahren zur Herstellung der Verbindungen der allgemeinen Formeln **Ia** und **Ib** 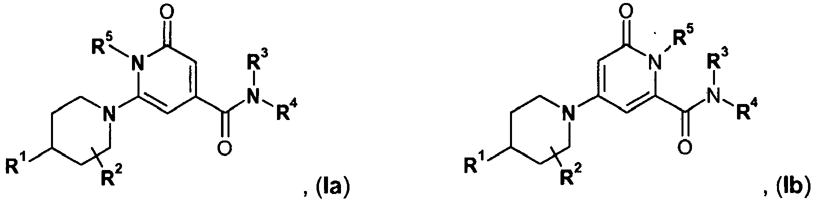 in der **R¹, R², R³, R⁴** und **R⁵** wie voranstehend erwähnt definiert sind.

Einige Methoden zur Herstellung der erfindungsgemäßen Verbindungen der allgemeinen Formel **Ia** sind in den folgenden Syntheseschemata und Beispielen dargestellt.

Die regioisomeren Verbindungen der allgemeinen Formel **Ib**, in denen **R¹, R², R³, R⁴** und **R⁵** wie voranstehend erwähnt definiert sind, können analog den nachstehend beschriebenen Verfahren hergestellt werden.

In einigen Fällen kann die Reihenfolge bei der Durchführung der Reaktionsschemata variiert werden, um die Reaktionen zu vereinfachen oder um unerwünschte Nebenprodukte zu verhindern. Die nachfolgenden Beispiele sind genannt, um die Erfindung vollständig verständlich zu machen. Die Beispiele sollen der Anschaulichkeit der Erfindung dienen und sollen die Erfindung in keinster Weise einschränken.

Die erfindungsgemäßen Verbindungen können gemäß den dargestellten Schemata und spezifischen Beispielen oder entsprechenden Modifikationen davon hergestellt werden. Von diesen Reaktionen können auch Abwandlungen eingesetzt werden, die einem Fachmann bekannt sind, hier aber nicht detailliert beschrieben sind. Die allgemeinen Verfahren zur Herstellung der erfindungsgemäßen Verbindungen erschließen sich einem Fachmann beim Anblick der folgenden Schemata.

Ausgangsverbindungen sind kommerziell verfügbar oder werden gemäß Verfahren hergestellt, die in der Literatur beschrieben sind, in dem Fachgebiet dem Fachmann bekannt sind oder wie sie hierin beschrieben sind. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach für den Fachmann geläufigen Methoden wieder abgespalten werden.

Bei den nachstehend beschriebenen Umsetzungen können gegebenenfalls vorhandene reaktive Gruppen wie Hydroxy-, Carboxy-, Amino-, Alkylamino-, Amid- oder Iminogruppen während der Umsetzung durch übliche Schutzgruppen geschützt werden, welche nach der Umsetzung wieder abgespalten werden.
Beispielsweise kommt
- als Schutzrest für eine Hydroxygruppe die Methoxy-, Benzyloxy-,Trimethylsilyl-, Acetyl-, Benzoyl-, tert.-Butyl-, Trityl-, Benzyl- oder Tetrahydropyranylgruppe,
- als Schutzreste für eine Carboxylgruppe die Trimethylsilyl-, Methyl-,Ethyl-, tert.-Butyl-, Benzyl- oder Tetrahydropyranylgruppe, und
- als Schutzreste für eine Amidgruppe die N-Methoxymethyl- (MOM), N-Benzyloxymethyl- (BOM), N-(Trimethylsilyl)ethoxymethyl (SEM), N-tert-Butyldimethylsiloxymethyl, N-tert-Butyldimethylsilyl- (TBDMS), N-Triisopropylsilyl- (TIPS), N-Benzyl-, N-4-Methoxybenzyl- (PMB), N-Triphenylmethyl- (Tr), N-tert-Butoxycarbonyl-(BOC), N-Benzyloxycarbonyl- (Cbz), N-Trimethylsilylethylsulfonyl- (SES)
- als Schutzrest für eine Amino-, Alkylamino- oder Iminogruppe die Acetyl-, Trifluoracetyl-, Benzoyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe,
in Betracht.

Weitere Schutzgruppen und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0°C und 100°C, vorzugsweise bei Temperaturen zwischen 10°C und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0°C und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0°C und 50°C, vorzugsweise jedoch bei Raumtemperatur, erfolgen.

Die Abspaltung einer Methoxygruppe erfolgt zweckmäßigerweise in Gegenwart von Bortribromid in einem Lösungsmittel wie Methylenchlorid bei Temperaturen zwischen -35°C und -25°C. Alternativ kann eine Methoxygruppe auch durch Brønsted-Säuren mit oder ohne Lösungsmittel gespalten werden. Bevorzugt ist Pyridin Hydrochlorid bei erhöhten Temperaturen ohne Lösungsmittel.

Die Abspaltung eines 2,4-Dimethoxybenzylrestes erfolgt vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder *n*-Butylamin in einem Lösungsmittel wie Methanol, Ethanol, Isopropanol, Toluol-Wasser oder Dioxan bei Temperaturen zwischen 20°C und 50°C.

Die Abspaltung eines Methoxymethyl-Rests kann in Gegenwart einer Säure wie konzentrierter Salzsäure in einem Lösungsmittel wie Dimethoxyethan durchgeführt werden. Alternativ kann eine Säure wie Trifluoressigsäure auch ohne Lösungsmittel eingesetzt werden.

Die Abspaltung eines N-(Trimethylsilyl)ethoxymethyl-Rests kann in Gegenwart von TBAF und 1,3-Dimethyl-3,4,5,6-Tetrahydro-2(1*H*)-Pyrimidon durchgeführt werden. Alternativ kann die SEM-Schutzgruppe auch mit einer Säure wie Chlorwasserstoff in einem organischen Lösungsmittel wie Dioxan oder Ethanol abgespalten werden.

Die Abspaltung eines Allyloxycarbonylrestes erfolgt durch Behandlung mit einer katalytischen Menge Tetrakis-(triphenyl-phosphin)-palladium(0) vorzugsweise in einem Lösungsmittel wie Tetrahydrofuran und vorzugsweise in Gegenwart eines Überschusses von einer Base wie Morpholin bei Temperaturen zwischen 0°C und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo-[2.2.2]octan bei Temperaturen zwischen 20°C und 70°C.

Die folgenden Verfahren zur Herstellung der erfindungsgemäßen Verbindungen der allgemeinen Formel **Ia**, in denen **R¹, R², R³, R⁴** und **R⁵** wie voranstehend erwähnt definiert sind, und ihrer Vorstufen haben sich besonders bewährt:

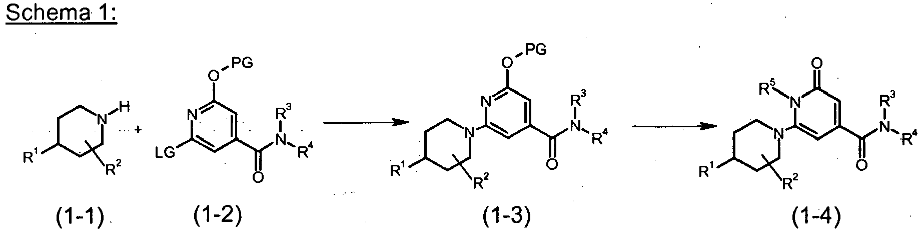

Die Herstellung einer Verbindung der allgemeinen Formel (1-4), in der **R¹, R², R³** und **R⁴** wie voranstehend erwähnt definiert sind und **R⁵** ein Wasserstoffatom darstellt, ist in Schema 1 gezeigt. Eine Verbindung der allgemeinen Formel (1-1), in der **R¹** und **R²** wie voranstehend erwähnt definiert sind, kann mit einer Verbindung der allgemeinen Formel (1-2), in der **R³** und **R⁴** wie voranstehend erwähnt definiert sind, **LG** eine Austrittsgruppe darstellt und **PG** eine Schutzgruppe bedeutet, umgesetzt werden. Als Austrittsgruppe **LG** können Halogenide, bevorzugt Chloride und Bromide, -SO₂CH₃, -OSO₂CH₃, -OSO₂C₆H₄-CH₃ oder -S-CH₃ (-S-CH₃ erfordert die weitere Umsetzung mit einem organischen Peroxid um in die eigentliche Austrittsgruppe überführt werden zu können) etc. fungieren, müssen aber nicht auf diese beschränkt sein. Ganz besonders bevorzugt ist die Verwendung von Chloriden. Schutzgruppen **PG** für die Hydroxy-Funktionalität sind dem Fachmann bekannt oder sind in der Literatur (T.W. Greene, P.G.M. Wuts, "Protective Groups in Organic Synthesis", Wiley, 1999) beschrieben. Ganz besonders bevorzugt ist die Methoxy-Schutzgruppe.

Die Reaktion kann in einem inerten Lösungsmittel unter Verwendung einer Hilfsbase in einem Temperaturbereich von 0°C bis zum Rückfluss des Lösungsmittels erfolgen. Die Reaktion wird in einem geeigneten, inerten Lösungsmittel, wie Tetrahydrofuran, Toluol, Xylol, Dialkylformamid (besonders bevorzugt Dimethylformamid), cyclische Amide (besonders bevorzugt N-Methylpyrrolidon), 1,4-Dioxan, Acetonitril oder in Lösungsmittelgemischen durchgeführt. Als geeignete Hilfsbasen können tertiäre Amine wie Triethylamin oder Ethyldiisopropylamin, Alkalimetall-Carbonate wie Kaliumcarbonat oder Natriumcarbonat, Natriumhydrid (NaH) oder Lithiumdiisopropylamid (LDA) verwendet werden. Dabei muss das verwendete inerte Lösungsmittel kompatibel sein mit der verwendeten Base. Bevorzugt wird die Reaktion in N-Methylpyrrolidon, bei Temperaturen zwischen Raumtemperatur und Rückfluss des Lösungsmittels in Gegenwart von Kaliumcarbonat als Hilfsbase durchgeführt.
Ausgehend von einer Verbindung der allgemeinen Formel (1-3), in der **R¹, R², R³** und **R⁴** wie voranstehend erwähnt definiert sind und **PG** eine Schutzgruppe darstellt, kann durch Etherspaltung eine Verbindung der allgemeinen Formel (1-4), in der **R¹, R², R³** und **R⁴** wie voranstehend erwähnt definiert sind und **R⁵** ein Wasserstoffatom darstellt, wie in Schema 1 gezeigt erfolgen. Ether lassen sich mit Brønsted-Säuren oder Lewis-Säuren spalten. Ganz besonders bevorzugt ist die Umsetzung von Verbindungen der allgemeinen Formel (1-3) mit Pyridin Hydrochlorid ohne Lösungsmittel bei erhöhten Temperaturen. Schutzgruppen **PG** für die Hydroxy-Funktionalität sind dem Fachmann bekannt oder sind in der Literatur (T.W. Greene, P.G.M. Wuts, "Protective Groups in Organic Synthesis", Wiley, 1999) beschrieben. Ganz besonders bevorzugt ist die Methoxy-Schutzgruppe.

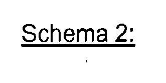

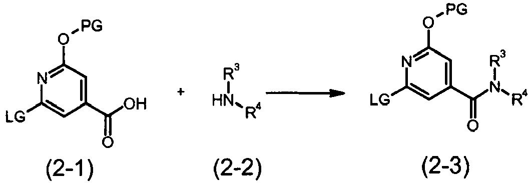

Verbindungen der allgemeinen Formel (2-3), in der **R³** und **R⁴** wie voranstehend erwähnt definiert sind, **LG** eine Austrittsgruppe und **PG** eine Schutzgruppe repräsentiert, können analog Schema 2 synthetisiert werden. Als Austrittsgruppe **LG** können Halogenide, bevorzugt Chloride und Bromide, -SO₂CH₃, -OSO₂CH₃, -OSO₂C₆H₄-CH₃ oder -S-CH₃ (-S-CH₃ erfordert die weitere Umsetzung mit einem organischen Peroxid um in die eigentliche Austrittsgruppe überführt werden zu können) etc. fungieren, müssen aber nicht auf diese beschränkt sein. Ganz besonders bevorzugt ist die Verwendung von Chloriden. Schutzgruppen **PG** für die Hydroxy-Funktionalität sind dem Fachmann bekannt oder sind in der Literatur (T.W. Greene, P.G.M. Wuts, "Protective Groups in Organic Synthesis", Wiley, 1999) beschrieben. Ganz besonders bevorzugt ist eine Schützung der Hydroxy-Funktionalität durch eine Methoxy-Schutzgruppe.

Carbonsäuren der allgemeinen Formel (2-1), in der **PG** ein Schutzgruppe und **LG** eine Austrittsgruppe repräsentieren, können mit Verbindungen der allgemeinen Formel (2-2), in der **R³** und **R⁴** wie voranstehend erwähnt definiert sind, unter Zuhilfenahme von Standard-Peptid-Kupplungsreagenzien und einer Base in einem inerten Lösungsmittel zu Amiden der allgemeinen Formel (2-3) umgesetzt werden (siehe z. B. Houben-Weyl, Methoden der Organischen Chemie, Bd. 15/2). Als inerte Lösungsmittel können Dimethylformamid, N-Methylpyrrolidon, Dimethoxyethan, Dichlormethan, Acetonitril oder Lösungsmittelgemische verwendet werden. Bevorzugtes Lösungsmittel ist Dimethylformamid. Geeignete Basen sind besonders Amin-Basen wie z.B. Triethylamin oder Diisopropylethylamin. Als Kupplungsreagenzien können beispielsweise 1*H*-Benzotriazol-1-yl-oxy-tri-pyrrolidino-phosphonium-hexafluorophosphat (PyBOP), Dicyclohexylcarbodiimid (DCC), Diisopropylcarbodiimid (DIC), Ethyl-(3-dimethylamino-propyl)-carbodiimid, O-(1*H*-Benzotriazol-1-yl)-N,N-N,N-tetramethyl-uronium-hexafluorphosphat (HBTU) oder -tetrafluorborat (TBTU) oder 1*H*-Benzotriazol-1-yl-oxy-tris-(dimethylamino)-phosphonium-hexafluorphosphat (BOP) verwendet werden. Besonders bevorzugt ist die Verwendung von TBTU. Die Aktivierung der Carboxylgruppe kann auch über ein entsprechendes Säureanhydrid oder Säurechlorid erfolgen. Die Umsetzung erfolgt im Allgemeinen in einem Temperaturbereich von -20°C bis zum Rückfluss des Lösungsmittels bei Normaldruck. Besonders bevorzugt ist die Verwendung von Diisopropylethylamin als Base und Dimethylformamid als Lösungsmittel.

Die Verbindungen der allgemeinen Formel (3-3), in denen **R¹, R², R³** und **R⁴** wie voranstehend erwähnt definiert sind und **R⁵** eine C₁₋₆-Alkylgruppe darstellt, können analog Schema 3 synthetisiert werden.

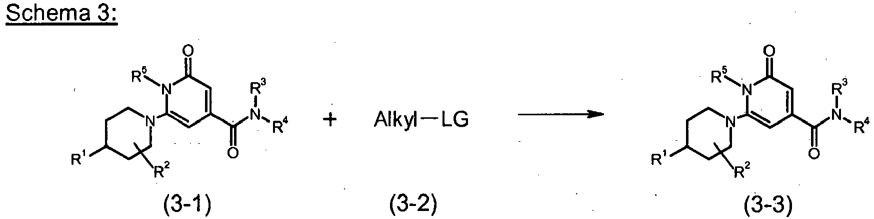

Eine Verbindung der allgemeinen Formel (3-1), in der **R¹, R², R³** und **R⁴** wie voranstehend erwähnt definiert sind und **R⁵** ein Wasserstoffatom darstellt, kann mit einer Verbindung der allgemeinen Formel (3-2), in der Alkyl eine C₁₋₆-Alkylgruppe und **LG** eine Austrittsgruppe darstellt, umgesetzt werden. Als Austrittsgruppe können Halogenide, bevorzugt Bromide und Iodide, -OSO₂CH₃, -OSO₂C₆H₄-CH₃, etc. fungieren, müssen aber nicht auf diese beschränkt sein. Ganz besonders bevorzugt ist die Verwendung von Iodiden. Ganz besonders bevorzugt ist die Verwendung von Methyljodid. Die Reaktion kann in einem inerten Lösungsmittel unter Verwendung einer Hilfsbase in einem Temperaturbereich von 0°C bis zum Rückfluss des Lösungsmittels erfolgen. Als inerte Lösungsmittel können Dimethylformamid, N-Methylpyrrolidon, Dimethylsulfoxid, Acetonitril oder Lösungsmittelgemische verwendet werden. Bevorzugtes Lösungsmittel ist Dimethylsulfoxid. Als geeignete Hilfsbasen können Alkalimetall-Carbonate wie Kaliumcarbonat, Natriumcarbonat oder Cäsiumcarbonat verwendet werden. Dabei muss das verwendete inerte Lösungsmittel kompatibel sein mit der verwendeten Base. Ganz besonders bevorzugt ist die Verwendung von Cäsiumcarbonat.

In einigen Fällen kann das Endprodukt weiter derivatisiert werden, z.B. durch Manipulation der Substituenten. Diese Manipulationen können unter anderem diejenigen sein, die dem Fachmann allgemein bekannt sind wie Oxidation, Reduktion, Alkylierung, Acylierung und Hydrolyse, müssen allerdings nicht auf diese beschränkt sein.

Die erfindungsgemäßen neuen Verbindungen der allgemeinen Formeln **Ia** und **Ib** können ein oder mehrere Chiralitätszentren enthalten. Sind beispielsweise zwei Chiralitätszentren vorhanden, dann können die Verbindungen in Form zweier diastereomerer Antipodenpaare auftreten. Die Erfindung umfasst die einzelnen Isomere ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formeln **Ia** und **Ib** fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (R)-(+)-I-Phenylethylamin, (S)-(-)-I-Phenylethylamin oder (S)-Brucin, entstehen.

Nach einem üblichen Verfahren zur Isomerentrennung wird das Racemat einer Verbindung der allgemeinen Formeln **Ia** und **Ib** mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formeln **Ia** und **Ib** fallender, diastereomerer Verbindungen wird auch dadurch erhalten, dass man die oben beschriebenen Synthesen mit jeweils einer geeigneten (R)- bzw. (S)-konfigurierten Reaktionskomponente durchführt.

Die neuen Verbindungen der allgemeinen Formeln **Ia** und **Ib** und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC Membranen (~20 µg Proteinmenge) werden für 180 Minuten bei Raumtemperatur mit 50 pM ¹²⁵I-Iodotyrosyl-Calcitonin-Gene-Related Peptide und ansteigenden Konzentrationen der Testsubstanzen in einem Gesamtvolumen von 250 µl inkubiert (Assay Puffer: 10 mM Tris, 50 mM NaCl, 5 mM MgCl₂, 1mM EDTA, pH=7.4). Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM BIBN4096BS während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test Kᵢ-Werte ≤ 50 µM.

### B. CGRP-Antagonismus in SK-N-MC-Zellen

SK-N-MC Zellen (∼1000 Zellen pro well) werden in Anwesenheit von steigenden Konzentrationen von CGRP und verschiedenen Konzentrationen der Testsubstanz für 30 Minuten inkubiert.

Die cAMP-Gehalte der Proben werden mittels AlphaScreen cAMP assay kit (Perkin Elmer) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁴ M.

Um zu zeigen, dass die Verbindungen der allgemeinen Formeln **Ia** und **Ib** mit unterschiedlichen Strukturelementen gute bis sehr gute CGRP-antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht, um spezifische Verbindungen hervorzuheben:

| **Beispiel** | **Kᵢ [nM]** |
|---|---|
| (2) | 480 |
| (3) | 8.4 |
| (4) | 3.8 |

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/-Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing Ionenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der allgemeinen Formeln **Ia** und **Ib** gemäß den obigen bevorzugten Ausführungsformen.

Besonders bevorzugt ist es, wenn die Verbindungen der allgemeinen Formeln **Ia** und **Ib** oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen. Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der allgemeinen Formeln **Ia** und **Ib** inhalativ verabreicht werden, besonders bevorzugt ist es, wenn die Verabreichung ein oder zweimal täglich erfolgt. Hierzu müssen die Verbindungen der allgemeinen Formeln **Ia** und **Ib** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### EXPERIMENTELLER TEIL

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.
Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Verwendete Laufmittelsysteme für DC:
- Laufmittel A: DCM/Cyclohexan/MeOH/NH₄OH = 70/15/15/2
- Laufmittel B: Petrolether/Ethylacetat = 2/1

Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX*^{™}*, 35-70 µm) verwendet.
Die angegebenen HPLC-Daten werden unter nachstehend angeführten Parametern und unter Verwendung der aufgeführten Säulen erhoben:

### Verwendete Säulen:

### (Säulentemperatur: 30 °C; Injektionsvolumen: 5 µL; Detektion bei 254 nm)

| | |
|---|---|
| S1 | Zorbax-Säule (Agilent Technologies), SB (Stable Bond) C18; 3.5 µm; 4.6 x 75 mm |
| S2 | Waters Sunfire, SB (Stable Bond) C18; 3.5 µm; 4.6 x 75 mm |
| S3 | Agilent Bonus C18; 5 µm, 4.6 x 75 mm |
| S4 | Zorbax-Säule (Agilent Technologies), SB (Stable Bond) C18; 1.8 µm; 3.0 x 30 mm |
| S5 | Zorbax-Säule (Agilent Technologies), SB (Stable Bond) C18; 5 µm; 4.6 x 75 mm |
| S6 | Waters Symmetry C18; 3.5 µm; 4.6 x 75 mm |
| S7 | Waters XBridge C18; 3.5 µm; 4.6 x 75 mm (basische Säule) |

### Verwendete Lösungsmittel:

- Für die Säulen S1 bis S6 (saure Bedingungen) wurden als Lösungsmittel verwendet:
   Lösungsmittel A: Wasser (mit 0.1 % Ameisensäure)
   Lösungsmittel B: Acetonitril (mit 0.1% Ameisensäure)
- Für die Säule S7 (basische Bedingungen) wurden folgende Lösungsmittel verwendet:
   Lösungsmittel A: Wasser (mit 0.1% NH₄OH)
   Lösungsmittel B: Acetonitril (mit 0.1% NH₄OH)
   (die prozentualen Angaben beziehen sich auf das Gesamtvolumen)

### Gradienten:

| Gradient (Fluss) | Zeit [min] | %A | %B |
|---|---|---|---|
| **G1** (0.8 mL/min) | 0.0 | 95 | 5 |
| | 8.0 | 50 | 50 |
| | 9.0 | 10 | 90 |
| | 10.0 | 10 | 90 |
| | 11.0 | 95 | 5 |
| **G2** (1.6 mL/min) | 0.00 | 95 | 5 |
| | 0.10 | 95 | 5 |
| | 1.75 | 5 | 95 |
| | 1.90 | 5 | 95 |
| | 1.95 | 95 | 5 |
| | 2.00 | 95 | 5 |
| **G3** (1.6 mL/min) | 0.00 | 95 | 5 |
| | 4.50 | 10 | 90 |
| | 5.00 | 10 | 90 |
| | 5.50 | 95 | 5 |
| **G4** (1.6 mL/min) | 0.00 | 95 | 5 |
| | 4.00 | 50 | 50 |
| | 4.50 | 10 | 90 |
| | 5.00 | 10 | 90 |
| | 5.50 | 95 | 5 |
| **G5** (1.6 mL/min) | 0.00 | 90 | 10 |
| | 4.50 | 10 | 90 |
| | 5.50 | 10 | 90 |
| **G6** (0.8 mL/min) | 0.0 | 95 | 5 |
| | 9.0 | 10 | 90 |
| | 10.0 | 10 | 90 |
| | 11.0 | 95 | 5 |
| **G7** (1.6 mL/min) | 0.00 | 95 | 5 |
| | 2.00 | 50 | 50 |
| | 2.25 | 10 | 90 |
| | 2.50 | 10 | 90 |
| | 2.75 | 95 | 5 |

### Methoden:

| Methode | Säule | Gradient |
|---|---|---|
| Methode A | S1 | G4 |
| Methode B | S2 | G4 |
| Methode C | S4 | G2 |
| Methode D | S6 | G4 |
| Methode E | S1 | G3 |
| Methode F | S3 | G3 |
| Methode G | S5 | G4 |
| Methode H | S1 | G5 |
| Methode K | S2 | G3 |
| Methode L | S1 | G2 |
| Methode M | S7 | G3 |
| Methode N | S2 | G1 |
| Methode O | S4 | G7 |

Bei präparativen HPLC-Reinigungen erfolgt die Sammlung der Produkte entweder massengesteuert oder über UV-Detektion. Die Produkt enthaltenden Fraktionen werden vereinigt und gefriergetrocknet. Für präparative HPLC-Trennungen können folgende Säulen verwendet werden:

| | |
|---|---|
| S8 | Agilent Zorbax SB C18, 50 x 150 mm, 5 µm |
| S9 | Agilent Zorbax Stable Bond, 50 x 140 mm, 7 µm |
| S10 | Waters Sunfire C18, 30 x 100 mm, 5 µm |
| S11 | Waters Symmetry 50 x 140 mm, 7 µm |
| S12 | Agilent Zorbax Stable Bond C18, 30 x 100 mm, 5 µm, |

Für die präparative HPLC-Trennung können folgende Lösungsmittelsysteme verwendet werden:
- Lösungsmittel A: Wasser (mit 0.1 % Ameisensäure)
   Lösungsmittel B: Acetonitril (mit 0.1% Ameisensäure)
- Lösungsmittel A: Wasser (mit 0.15% Ameisensäure)
   Lösungsmittel B: Acetonitril (mit 0.15% Ameisensäure)
- Lösungsmittel A: Wasser (mit 0.3% Ameisensäure)
   Lösungsmittel B: Acetonitril
- Lösungsmittel A: Wasser (mit 0.3% Ameisensäure)
   Lösungsmittel B: Acetonitril (mit 0.3% Ameisensäure)
- Lösungsmittel A: Wasser (mit 0.1% NH₄OH)
   Lösungsmittel B: Acetonitril (mit 0.1% NH₄OH)
   (die prozentualen Angaben beziehen sich auf das Gesamtvolumen)

Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- AcOH: Essigsäure
- BINAP: 2,2'-Bis-(diphenylphosphino)-1,1'-binaphtyl
- BOC: *tert*.-Butyloxycarbonyl
- CDI: 1,1'-Carbonyldiimidazol
- Cyc: Cyclohexan
- DC: Dünnschichtchromatögraphie
- DCM: Dichlormethan
- DIPE: Diisopropylether
- DIPEA: Diisopropylethylamin
- DMF: *N,N*-Dimethylformamid
- DMSO: Dimethylsulfoxid
- dppf: 1,1'-Bis-(diphenylphosphino)ferrocen
- d. Th.: der Theorie
- EI: Elektronenstoß-Ionisation (bei MS)
- Eq: Äquivalent
- ESI: Electron Spray Ionisation (bei MS)
- EtOAc: Essigsäureethylester
- EtOH: Ethanol
- E-Wasser: entionisiertes Wasser
- h: Stunde(n)
- HCl: Chlorwasserstoff
- HPLC: High Performance Liquid Chromatography
- HPLC-MS: HPLC gekoppelte Massenspektrometrie
- i.vac.: in vacuo (im Vakuum)
- konz.: konzentriert
- M: molar
- mmol: Millimol
- mL: Milliliter
- µL: Mikroliter
- MeOH: Methanol
- MS: Massenspektrometrie
- MW: Molekulargewicht [g/mol]
- NaOAc: Natriumacetat
- NaOH: Natriumhydroxid
- NH₄OH: Ammoniumhydroxid (wässrige Ammoniak-Lösung, 30%)
- NMP: *N*-Methylpyrrolidon
- PE: Petrolether
- quant.: quantitativ
- R_{f}: Retentionsfaktor (bei DC)
- Rₜ: Retentionszeit (bei HPLC)
- RT: Raumtemperatur
- TBTU: O-(Benzotriazol-1-yl)-*N,N,N*'*,N*'-tetramethyluronium-tetrafluoroborat
- TEA: Triethylamin
- TFA: Trifluoressigsäure
- THF: Tetrahydrofuran
- ULTS: Umluft-Trockenschrank
- XantPhos: 4,5-Bis(Diphenylphosphino)-9,9-dimethylxanthen
- XPhos: 2-Dicyclohexylphosphino-2',4',6'-triisopropylbiphenyl

### Herstellung der Ausgangsverbindungen:

### Intermediat 1a:

### 1-Piperidin-4-yl-1,3-dihydroimidazo[4,5-b]pyridin-2-on-Dihydrochlorid

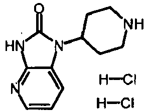

Diese Verbindung und deren Vorstufen wurden wie in der internationalen Anmeldung WO 2005/013894 beschrieben synthetisiert.

| | |
|---|---|
| ESI-MS: | m/z = 219 (M+H)⁺ |
| R_{f}: | 0.11 (Kieselgel, DCM/MeOH/NH₄OH = 80:20:2) |

### Intermediat 1b:

### 1-Piperidin-4-yl-1,3-dihydroimidazo[4,5-b]pyridin-2-on

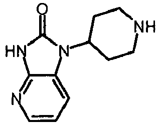

### Stufe 1: 4-(2-Chlor-pyridin-3-yl-amino)-piperidin-1-carbonsäurebenzylester

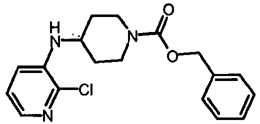

Zu 930 g (3.99 mol) *N*-Benzyloxycarbonyl-4-piperidon und 466 g (3.63 mol) 2-Chlor-3-aminopy idin in 9.5 L Isopropylacetat wurden bei ca. 15°C 560 mL (7.25 mol) TFA zugetropft. 922 g (4.35 mol) Natriumtriacetoxyborhydrid wurden portionsweise zugegeben. Es wurde bis zur Vollständigkeit der Reaktion nachgerührt. Bei RT wurde die Reaktionsmischung mit 860 mL Natronlauge (2 mol/L) versetzt. Die organische Phase wurde abgetrennt, mit 5 L Wasser gewaschen und eingeengt.

| | |
|---|---|
| Ausbeute: | 1250 g (roh, quant.) |
| ESI-MS: | m/z = 346 (M+H)⁺ |

### Stufe 2: 4-[1-(2-Chlor-pyridin-3-yl)-ureido]-piperidin-1-carbonsäurebenzylester

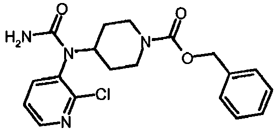

530 mL (6.1 mol) Chlorsulfonylisocyanat wurden in 6 L THF vorgelegt und auf -15°C abgekühlt. Zu diesem Gemisch wurde anschließend eine Lösung von 1.25 kg (3.63 mol) 4-(2-Chlor-pyridin-3-yl-amino)-piperidin-1-carbonsäurebenzylester in 7 L THF innerhalb einer Stunde so zugetropft, dass die Temperatur der Reaktionsmischung -7°C nicht überstieg. Es wurde 90 Minuten bei ca. -8°C nachgerührt und anschließend 700 mL Wasser innerhalb von 30 Minuten zugetropft. Die Mischung wurde 30 Minuten bei ca. 10°C nachgerührt und anschließend mit 8.1 L Natronlauge (2 mol/L) langsam versetzt. Das Reaktionsgemisch wurde anschließend auf 50°C erwärmt und die Phasen getrennt. Die organische Phase wurde mit 2 L Wasser gewaschen. Danach wurden aus der organischen Phase 10 L Lösemittel abdestilliert, 15 L Butylacetat zum Rückstand zugegeben und hiervon nochmals 8 L abdestilliert. Durch langsames Abkühlen auf 0°C wurde das Produkt kristallisiert. Der Niederschlag wurde abgesaugt, mit 2L Butylacetat gewaschen und bei 40°C getrocknet.

| | |
|---|---|
| Ausbeute: | 1108 g (79% d. Th.) |
| ESI-MS: | m/z = 389/391 (M+H)⁺ |

### Stufe 3: 4-(2-Oxo-2,3-dihydro-imidazo[4,5-b]pyridin-1-yl)-piperidin-1-carbonsäure-Benzylester

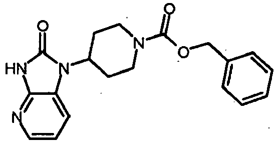

1108 g (2.85 mol) 4-[1-(2-Chlor-pyridin-3-yl)-ureido]-piperidin-1-carbonsäurebenzylester wurden mit 720 g (8.57 mol) Natriumhydrogencarbonat in 14.5 L tert-Amylalkohol unter Rückfluss erhitzt. Hierbei wurden 3 L Lösemittel abdestilliert. Das Reaktionsgemisch wurde auf 35°C abgekühlt und mit 11 mL Wasser versetzt. Anschließend wurden 13 g (0.058 mol) Palladiumacetat und 49 g (0.115 mol) 1,4-Bis-(diphenylphosphino)-butan (DPPB) zugegeben und auf Rückflusstemperatur erhitzt. Es wurde bei 100°C bis zur Vollständigkeit der Reaktion nachgerührt, auf RT abgekühlt und 7.5 L Wasser zugegeben. Die organische Phase wurde abgetrennt, mit 5 L Wasser gewaschen und anschließend eingeengt. Der ölige Rückstand wurde zweimal mit je 3 L Isopropylacetat versetzt und abdestilliert. Anschließend wurde der Rückstand in 7 L Isopropylacetat heiß gelöst und langsam auf Raumtemperatur abgekühlt. Der auskristallisierte Feststoff wurde abgesaugt, mit je 2 L Isopropylacetat und tert.-Butyl-methylether gewaschen und bei 50°C getrocknet.

| | |
|---|---|
| Ausbeute: | 690 g (69% d. Th.) |
| ESI-MS: | m/z = 353 (M+H)⁺ |

### Stufe 4: 1-Piperidin-4-yl-1,3-dihydro-imidazo[4,5-b]pyridin-2-on

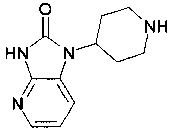

690 g (1.96 mol) 4-(2-Oxo-2,3-dihydro-imidazo[4,5-b]pyridin-1-yl)-piperidin-1-carbonsäure-benzylester wurden in 5.4 L Methanol gelöst und unter Zusatz von 46 g Pd/C (10%ig; 6.6 Gew.%) bei 60°C und einem Wasserstoffdruck von 60 psi bis zur vollständigen Wasserstoffaufnahme hydriert. Der Katalysator wurde abfiltriert. Vom Filtrat wurden 4 L Methanol abdestilliert. Es wurden 2 L Methylcyclohexan zugegeben und weitere 1.5 L Lösemittel abdestilliert. Die so erhaltene Suspension wurde abgesaugt, der Rückstand mit Methylcyclohexan gewaschen und bei 40°C getrocknet.

| | |
|---|---|
| Ausbeute: | 446 g (100% d. Th.) |
| ESI-MS: | m/z = 219 (M+H)⁺ |

### Intermediat 2:

### 7-Methoxy-3-piperidin-4-yl-1,3,4,5-tetrahydro-1,3-benzodiazepin-2-on

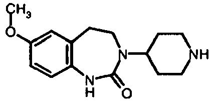

### Stufe 1: (5-Methoxy-2-nitrophenyl)-acetonitril

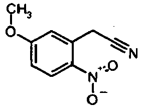

Zu einer Lösung aus 13.2 g (86.0 mmol) 4-Nitroanisol und 18.0 g (107 mmol) 4-Chlorphenoxyacetonitril in 50 mL DMF wurden 24.0 g (214 mmol) Kalium-*tert*-butylat in 100 mL DMF langsam zugetropft. Der Reaktionsansatz wurde 30 min bei -10°C gerührt und anschließend in 300 g einer 1:1 Mischung aus konz. HCl und Eis gegossen. Nach Extraktion mit EtOAc wurde die organische Phase mit Wasser gewaschen, getrocknet und i.vac. eingeengt. Der Rückstand wurde mit einer 1:1 Mischung PE/EtOAc behandelt und das auskristallisierte Produkt abgesaugt. Nach dem Nachwaschen mit einer 1:1 Mischung Petrolether/EtOAc wurden die Kristalle an der Luft getrocknet.

| | |
|---|---|
| Ausbeute: | 6.5 g (39% d. Th.) |
| ESI-MS: | m/z = 210 (M+NH₄)⁺ |
| R_{f}: | 0.45 (Kieselgel; PE/EtOAc = 1:1) |

### Stufe 2: 2-(5-Methoxy-2-nitrophenyl)-ethylamin

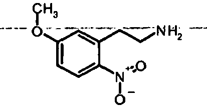

Unter einer Stickstoffatmosphäre wurden bei RT zu 12.6 g (65.7 mmol) (5-Methoxy-2-nitrophenyl)-acetonitril in 380 mL THF langsam 200 mL (200 mmol) einer 1M Boran in THF Lösung zugetropft. Der Reaktionsansatz wurde 2 h unter Rückfluss gekocht. Nach dem Erkalten wurden innerhalb von 20 min 30 mL Methanol zugetropft. Dabei wurde mit einem Eisbad die Temperatur bei 10°C bis 20°C gehalten. Man ließ den Reaktionsansatz 30 min bei RT nachrühren und tropfte im Anschluss daran innerhalb von 30 min 45 mL einer 2M wässrigen HCl-Lösung hinzu. Das Reaktionsgemisch wurde i.vac. einrotiert. Der Rückstand wurde mit Wasser auf ca. 200 mL verdünnt und mit 200 mL EtOAc extrahiert. Die wässrige Phase wurde mit einer 15%igen (w/v) wässrigen Kaliumcarbonat-Lösung alkalisch gestellt und über Nacht mit einem Rotations-Perforator nach Ludwig (Fa. Normag) mit Diethylether kontinuierlich extrahiert. Das organische Extrakt wurde bis zur Trockene einrotiert.

| | |
|---|---|
| Ausbeute: | 9.98 g (77% d. Th.) |
| ESI-MS: | m/z = 197 (M+H)⁺ |
| Rₜ(HPLC): | 2.1 min (Methode E) |

### Stufe 3: (1-Benzylpiperidin-4-yl)-[2-(5-methoxy-2-nitrophenyl)-ethyl]-amin

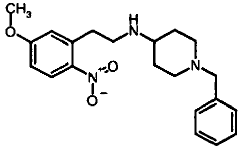

Unter einer Stickstoffatmosphäre wurde in einem Eisbad ein Gemisch aus 9.98 g (50.9 mmol) 2-(5-Methoxy-2-nitrophenyl)-ethylamin, 9.80 mL (54.9 mmol) N-Benzylpiperidon und 6.30 mL (114 mmol) Essigsäure in 270 mL Dichlormethan auf 0°C gekühlt. Bei dieser Temperatur wurden 14.2 g (67.0 mmol) Natriumtriacetoxyborhydrid portionsweise innerhalb von 20 min zugegeben. Man ließ den Reaktionsansatz weitere 4 h bei 0°C nachrühren und über Nacht auf RT aufwärmen. Anschließend wurde der Ansatz mit 400 mL einer 15%igen (w/v) wässrigen Kaliumcarbonat-Lösung versetzt und 1 h bei RT nachgerührt. Die organische Phase wurde abgetrennt, getrocknet und einrotiert.

| | |
|---|---|
| Ausbeute: | 18.8 g (quantitativ) |
| ESI-MS: | m/z = 370 (M+H)⁺ |
| Rₜ(HPLC): | 1.9 min (Methode E) |

### Stufe 4: [2-(2-Amino-5-methoxyphenyl)-ethyl]-(1-benzylpiperidin-4-yl)-amin

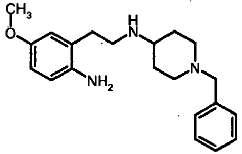

26.0 g (70.3 mmol) (1-Benzylpiperidin-4-yl)-[2-(5-methoxy-2-nitrophenyl)-ethyl]-amin wurden mit 5.00 g (2.45 mmol) Rhodiumkohle (5%, wasserfeucht) in 350 mL Methanol in einer 3 bar Wasserstoffatmosphäre 3 h bei RT hydriert. Der Katalysator wurde abgesaugt und die Lösung einrotiert. Der Rückstand wurde ohne weitere Aufreinigung sofort weiter umgesetzt.

| | |
|---|---|
| Ausbeute: | 23.9 g (quantitativ) |
| Rₜ(HPLC): | 0.99 min (Methode A) |

### Stufe 5: 3-(1-Benzylpiperidin-4-yl)-7-methoxy-1,3,4,5-tetrahydro-1,3-benzodiazepin-2-on

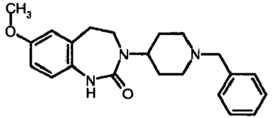

Zu 23.9 g (70.3 mmol) [2-(2-Amino-5-methoxyphenyl)-ethyl]-(1-benzylpiperidin-4-yl)-amin in 175 mL DMF wurden 35.0 g (216 mmol) *N,N'*-Carbonyldiimidazol zugegeben und die Mischung 2 h bei 100°C gerührt. Der Reaktionsansatz wurde auf ca. 1 kg Eiswasser gegossen und über Nacht gerührt. Das ausgefallene Produkt wurde abgesaugt, mit Wasser gewaschen und getrocknet. Der Rückstand wurde mit DIPE verrührt und abgesaugt. Das Festprodukt wurde mit DIPE nachgewaschen und getrocknet.

| | |
|---|---|
| Ausbeute: | 21.6 g (84% d. Th.) |
| ESI-MS: | m/z = 366 (M+H)⁺ |
| Rₜ(HPLC): | 2.12 min (Methode E) |

### Stufe 6: 7-Methoxy-3-piperidin-4-yl-1,3,4,5-tetrahydro-1,3-benzodiazepin-2-on

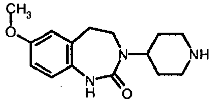

Eine Mischung aus 21.6 g (59.2 mmol) 3-(1-Benzylpiperidin-4-yl)-7-methoxy-1,3,4,5-tetrahydro-1,3-benzodiazepin-2-on und 2.5 g Palladium auf Kohle (10%) in 300 mL Methanol wurde in einer 3 bar Wasserstoffatmosphäre bei 50 °C bis zur vollständigen Umsetzung hydriert. Der Katalysator wurde abgesaugt und die Mutterlauge einrotiert. Der Rückstand wurde mit DIPE verrieben, abgesaugt, mit DIPE nachgewaschen und getrocknet.

| | |
|---|---|
| Ausbeute: | 13.2g(81%d.Th.) |
| ESI-MS: | m/z = 276 (M+H)⁺ |
| Rₜ(HPLC): | 0.73 min (Methode L) |

### Intermediat 3:

### (2-Chlor-6-methoxypyridin-4-yl)-(5-fluor-2,3-dihydroindol-1-yl)-methanon

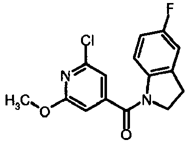

Zu 0.500 g (2.67 mmol) 2-Chlor-6-methoxyisonicotinsäure, 0.366 g (2.67 mmol) 5-Fluor-indolin und 0.421 mL (3.00 mmol) Triethylamin in 10.0 mL DMF wurde bei RT 0.965 g (3.00 mmol) TBTU hinzugegeben. Der Ansatz wurde 2 h bei RT gerührt und anschließend mittels präparativer HPLC aufgereinigt. Die Produkt enthaltenden Fraktionen wurden vereinigt und i.vac. eingeengt.

| | |
|---|---|
| Ausbeute: | 0.700 g (86% d. Th.) |
| ESI-MS: | m/z = 307 / 309 (M+H)⁺ (CI) |
| Rₜ(HPLC): | 1.60 min (Methode C) |

### Intermediat 4:

### 5-Fluor-3,3-dimethyl-2,3-dihydro-1H-indol

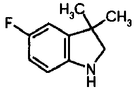

### Stufe 1: 1-Acetyl-5-fluor-1,3-dihydroindol-2-on

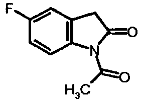

Bei 170°C wurden 3.0 g (20 mmol) 5-Fluorindolinon in 10 mL (98 mmol) Acetanhydrid für 3 h gerührt. Nach dem Abkühlen auf RT wurde der Ansatz auf 200 mL Eiswasser gegossen, die ausgefallene Substanz abgesaugt und mit 100 mL Wasser nachgewaschen. Der Feststoff wurde aus Wasser und Ethanol umkristallisiert. Das ausgefallene Produkt wurde abgesaugt, mit Wasser nachgewaschen und i.vac. getrocknet.

| | |
|---|---|
| Ausbeute: | 2.4 g (63 % d.Th.) |
| ESI-MS: | m/z = 192 (M+H)⁺ |
| Rₜ (HPLC): | 1.2 min (Methode C) |

### Stufe 2: 1-Acetyl-5-fluor-3,3-dimethyl-1,3-dihydroindol-2-on

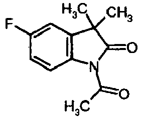

Bei 0°C bis 5°C wurde unter einer Argonatmosphäre zu 2.40 g (12.4 mmol) 1-Acetyl-5-fluor-1,3-dihydroindol-2-on in 30 mL DMF portionsweise 1.14 g (26.0 mmol) Natriumhydrid (55% in Mineralöl) zugegeben und 1 h nachgerührt. Anschließend wurden 1.91 mL (31.0 mmol) Methyliodid zugetropft und über Nacht bei RT gerührt. Der Reaktionsansatz wurde auf Wasser gegossen und die ausgefallene Substanz abgesaugt. Der Feststoff wurde mit Wasser nachgewaschen und i.vac. getrocknet.

| | |
|---|---|
| Ausbeute: | 2.1 g (76 % d.Th.) |
| ESI-MS: | m/z = 222 (M+H)⁺ |
| Rₜ(HPLC): | 1.48 min (Methode C) |

### Stufe 3: 5-Fluor-3,3-dimethyl-1,3-dihydroindol-2-on

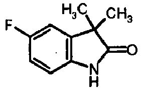

2.10 g (9.49 mmol) 1-Acetyl-5-fluor-3,3-dimethyl-1,3-dihydro-indol-2-on in 20 mL Isopropanol wurden mit 50 mL einer wässrigen 6M HCl-Lösung 1 h unter Rückfluss gekocht. Nach dem Abkühlen wurde das Isopropanol i.vac. entfernt. Der Rückstand wurde mit Wasser verdünnt und mit Eis gekühlt. Die ausgefallene Substanz wurde abgesaugt und mit Wasser nachgewaschen. Der Feststoff wurde i.vac. getrocknet.

| | |
|---|---|
| Ausbeute: | 1.40 g (82 % d.Th.) |
| ESI-MS: | m/z = 180 (M+H)⁺ |
| Rₜ(HPLC): | 1.14 min (Methode C) |

### Stufe 4: 5-Fluor-3,3-dimethyl-2,3-dihydro-1H-indol

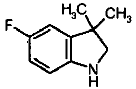

Unter einer Argonatmosphäre wurde zu 1.40 g (7.81 mmol) 5-Fluor-3,3-dimethyl-1,3-di-hydroindol-2-on in 50 mL THF eine Lösung aus 9.30 mL (9.30 mmol) einer 1 M Lösung Lithiumaluminiumhydrid in THF und 10 mL THF langsam zugetropft. Anschließend wurde der Reaktionsansatz für 1 h auf 70°C erwärmt. Nach dem Erkalten wurden 2 mL Wasser hinzugefügt. Die Lösung wurde über Natriumsulfat getrocknet und abfiltriert. Das Lösungsmittel wurde i.vac. entfernt.

| | |
|---|---|
| Ausbeute: | 1.30 g (quant.) |
| ESI-MS: | m/z = 166 (M+H)⁺ |
| Rₜ (HPLC): | 0.75 min (Methode C) |

### Intermediat 5:

### (2-Chlor-6-methoxypyridin-4-yl)-(5-fluor-3,3-dimethyl-2,3-dihydroindol-1-yl)-methanon

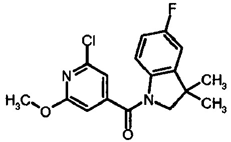

Diese Verbindung wurde analog zu (2-Chlor-6-methoxypyridin-4-yl)-(5-fluor-2,3-dihydro-indol-1-yl)-methanon aus 0.500 g (2.67 mmol) 2-Chlor-6-methoxyisonicotinsäure, 0.439 g (2.66 mmol) 5-Fluor-3,3-dimethyl-2,3-dihydro-1H-indol und 0.421 mL (3.00 mmol) Triethylamin in 10.0 mL DMF erhalten.

| | |
|---|---|
| Ausbeute: | 0.600 g (67% d.Th.) |
| ESI-MS: | m/z = 335 / 337 (M+H)⁺ (CI) |
| Rₜ(HPLC): | 1.73 min (Methode C) |

### Intermediat 6:

### 1-[4'-(5-Fluor-2,3-dihydroindol-1-carbonyl)-6'-methoxy-3,4,5,6-tetrahydro-2H-[1,2']bi-pyridinyl-4-yl]-1,3-dihydroimidazo[4,5-b]pyridin-2-on

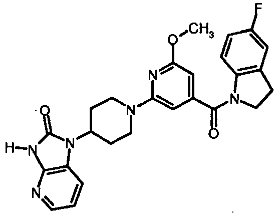

Ein Gemisch aus 0.361 g (1.24 mmol) 1-Piperidin-4-yl-1,3-dihydroimidazo[4,5-b]pyridin-2-on Dihydrochlorid, 0.380 mg (1.24 mmol) (2-Chlor-6-methoxypyridin-4-yl)-(5-fluor-2,3-dihydroindol-1-yl)-methanon und 514 mg (3.72 mmol) Kaliumcarbonat in 3.0 mL NMP wurde 8 h bei 130°C gerührt. Nach Abkühlen des Reaktionsgemisches auf RT wurde der ausgefallene Niederschlag abfiltriert und mittels präparativer HPLC aufgereinigt. Die Produkt enthaltenden Fraktionen wurden vereinigt und i.vac. eingeengt.

| | |
|---|---|
| Ausbeute: | 0.090 g (15% d. Th.) |
| ESI-MS: | m/z = 489 (M+H)⁺ |
| Rₜ(HPLC): | 1.50 min (Methode C) |

### Intermediat 7:

### 3-[4'-(5-Fluor-2,3-dihydroindol-1-carbonyl)-6'-methoxy-3,4,5,6-tetrahydro-2H-1,2'-bi-pyridinyl-4-yl]-7-methoxy-1,3,4,5-tetrahydro-1,3-benzodiazepin-2-on

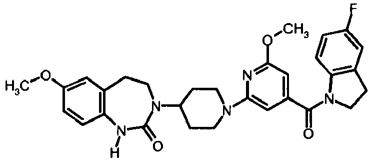

Ein Gemisch aus 0.633 g (2.30 mmol) 7-Methoxy-3-piperidin-4-yl-1,3,4,5-tetrahydro-1,3-benzodiazepin-2-on, 0.700 mg (2.28 mmol) (2-Chlor-6-methoxypyridin-4-yl)-(5-fluor-2,3-dihydroindol-1-yl)-methanon und 954 mg (6.90 mmol) Kaliumcarbonat in 5.0 mL NMP wurde 8 h bei 130°C gerührt. Nach Abkühlen des Reaktionsgemisches auf RT wurde der ausgefallene Niederschlag abfiltriert und mittels präparativer HPLC aufgereinigt. Die Produkt enthaltenden Fraktionen wurden vereinigt und i.vac. eingeengt. Der erhaltene Rückstand wurde in DMF digeriert und das als Feststoff verbliebene Produkt abgesaugt und i.vac. getrocknet. Die Mutterlauge wurde nochmals mittels präparativer HPLC aufgereinigt, die Produkt enthaltenden Fraktionen vereinigt und i.vac. eingeengt. Der Rückstand wurde mit Ethanol verrieben, das Festprodukt abgesaugt und i.vac. getrocknet.

| | |
|---|---|
| Ausbeute: | 0.490 g (39% d. Th.) |
| ESI-MS: | m/z = 546 (M+H)⁺ |
| Rₜ(HPLC): | 1.50 min (Methode C) |

### Intermediat 8:

### 1-[4'-(5-Fluor-3,3-dimethyl-2,3-dihydroindol-1-carbonyl)-6'-methoxy-3,4,5,6-tetrahydro-2H-[1,2']bipyridinyl-4-yl]-1,3-dihydroimidazo[4,5-b]pyridin-2-on

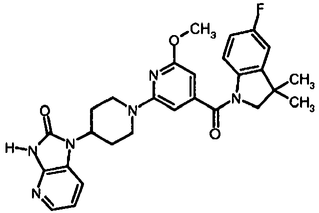

Diese Verbindung wurde analog zu 1-[4'-(5-Fluor-2,3-dihydroindol-1-carbonyl)-6'-methoxy-3,4,5,6-tetrahydro-2*H*-[1,2']bipyridinyl-4-yl]-1,3-dihydroimidazo[4,5-*b*]pyridin-2-on aus 262 mg (0.900 mmol) 1-Piperidin-4-yl-1,3-dihydroimidazo[4,5-b]pyridin-2-on Dihydrochlorid, 300 mg (0.90 mmol) (2-Chlor-6-methoxypyridin-4-yl)-(5-fluor-3,3-dimethyl-2,3-di-hydroindol-1-yl)-methanon und 373 mg (2.70 mmol) Kaliumcarbonat in 3.0 mL NMP erhalten.

| | |
|---|---|
| Ausbeute: | 40 mg (9% d. Th.) |
| ESI-MS: | m/z =517 (M+H)⁺ |
| Rₜ(HPLC): | 1.62 min (Methode C) |

### Intermediat 9:

### 1-[4'-(4,5-Difluor-2,3-dihydro-indol-1-carbonyl)-6'-methoxy-3,4,5,6-tetrahydro-2H-[1,2']bipyridinyl-4-yl]-1,3-dihydro-imidazo[4,5-b]pyridin-2-on

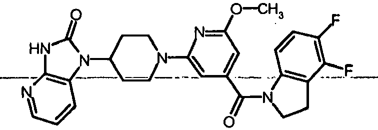

### Stufe 1: (2-Chlor-6-methoxy-pyridin-4-yl)-(4,5-difluor-2,3-dihydro-indol-1-yl)-methanon

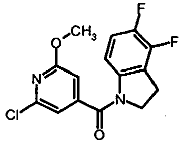

Zu 0.685 g (3.65 mmol) 2-Chlor-6-methoxyisonicotinsäure, 0.700 g (3.65 mmol) 4,5-Fluorindolin-dihydrochlorid und 1.12 mL (8.00 mmol) Triethylamin in 10.0 mL DMF wurden bei RT 1.22 g (3.80 mmol) TBTU gegeben. Der Ansatz wurde 2 h bei RT gerührt und anschließend auf 200 mL Kaliumcarbonatlösung (wässrig, 7%) gegossen. Der ausgefallene Niederschlag wurde abgesaugt, mit Wasser gewaschen und i. vac. getrocknet.

| | |
|---|---|
| Ausbeute: | 1.05 g (89% d. Th.) |
| ESI-MS: | m/z = 325 / 327 (M+H)⁺ (CI) |
| Rₜ(HPLC): | 1.66 min (Methode C) |

### Stufe 2: 1-[4'-(4,5-Difluor-2,3-dihydro-indol-1-carbonyl)-6'-methoxy-3,4,5,6-tetrahydro-2H-[1,2']bipyridinyl-4-yl]-1,3-dihydro-imidazo[4,5-b]pyridin-2-on

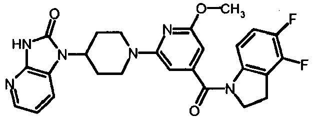

0.982 g (4.50 mmol) 1-Piperidin-4-yl-1,3-dihydroimidazo[4,5-b]pyridin-2-on und 500 mg (1.54 mmol) (2-Chlor-6-methoxy-pyridin-4-yl)-(4,5-difluor-2,3-dihydro-indol-1-yl)-methanon in 3.0 mL NMP wurden 12 h bei 130°C gerührt. Nach Abkühlen des Reaktionsgemisches auf RT wurde der ausgefallene Niederschlag abfiltriert und mittels präparativer HPLC aufgereinigt. Die Produkt enthaltenden Fraktionen wurden vereinigt und lyophilisiert.

| | |
|---|---|
| Ausbeute: | 0.250 g (32% d. Th.) |
| ESI-MS: | m/z = 507 (M+H)⁺ |
| Rₜ(HPLC): | 1.59 min (Methode C) |

### Intermediat 10:

### 1-[6'-(5-Fluor-2,3-dihydro-indol-1-carbonyl)-2'-methoxy-3,4,5,6-tetrahydro-2H-[1,4']bi-pyridinyl-4-yl]-1,3-dihydro-imidazo[4,5-b]pyridin-2-on

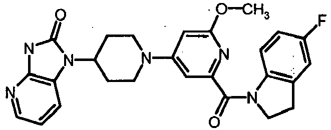

### Stufe 1: (4-Chlor-6-methoxy-pyridin-2-yl)-(5-fluor-2,3-dihydro-indol-1-yl)-methanon

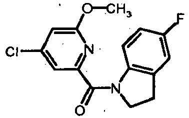

550 mg (2.93 mmol) 4-Chlor-6-methoxy-pyridin-2-carbonsäure, 411 mg (3.00 mmol) 5-Fluorindolin, 1.06 g (3.30 mmol) TBTU und 927 µL (6.60 mmol) Triethylamin in 5.00 mL DMF wurden 3 h bei RT gerührt. Der Reaktionsansatz wurde mittels. HPLC aufgereinigt. Die produktenthaltenden Fraktionen wurden vereinigt und am Rotationsverdampfer eingeengt.

| | |
|---|---|
| Ausbeute: | 450 mg (50% d. Th.) |
| ESI-MS: | m/z = 307 / 309 (M+H)⁺ (CI) |
| Rₜ(HPLC): | 1.7 min (Methode C) |

### Stufe 2: 1-[6'-(5-Fluor-2,3-dihydro-indol-1-carbonyl)-2'-methoxy-3,4,5,6-tetrahydro-2H-[1,4']bipyridinyl-4-yl]-1,3-dihydro-imidazo[4,5-b]pyridin-2-on

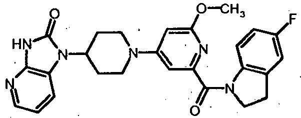

436 mg (2.00 mmol) 1-Piperidin-4-yl-1,3-dihydro-imidazo[4,5-b]pyridin-2-on und 200 mg (0.652 mmol) (4-Chlor-6-methoxy-pyridin-2-yl)-(5-fluor-2,3-dihydro-indol-1-yl)-methanon in 2 mL NMP würden über Nacht bei 120 °C gerührt. Der Reaktionsansatz wurde mittel präparativer HPLC aufgereinigt. Die produktenthaltenden Fraktionen wurden vereinigt und am Rotationsverdampfer eingeengt.

| | |
|---|---|
| Ausbeute: | 62 mg (20% d. Th.) |
| ESI-MS: | m/z = 487 (M-H)- |
| Rₜ(HPLC): | 1.7 min (Methode C) |

### Herstellung der Endverbindungen:

### Beispiel 1:

### 4'-(5-Fluor-2,3-dihydroindol-1-carbonyl)-4-(2-oxo-2,3-dihydroimidazo[4,5-b]pyridin-1-yl)-3,4,5,6-tetrahydro-2H,1'H-[1,2']bipyridinyl-6'-on

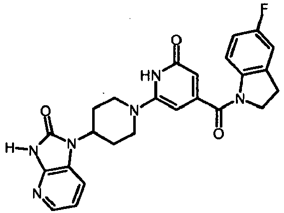

Ein gut gerührtes Gemisch aus 20 mg (0.041 mmol) 1-[4'-(5-Fluor-2,3-dihydroindol-1-carbonyl)-6'-methoxy-3,4,5,6-tetrahydro-2*H*-[1,2']bipyridinyl-4-yl]-1,3-dihydroimidazo-[4,5-*b*]pyridin-2-on und 100 mg (0.865 mmol) Pyridinhydrochlorid wurde 7 min mit dem Heißluftfön in der Schmelze gehalten. Nach dem Abkühlen der Reaktion wurde der Ansatz in DMF gelöst und mittels präparativer HPLC aufgereinigt. Die Produkt enthaltenden Fraktionen wurden vereinigt und lyophilisiert.

| | |
|---|---|
| Ausbeute: | 11 mg (57% d. Th.) |
| ESI-MS: | m/z = 476 (M+H)⁺ |
| Rₜ(HPLC): | 1.17 min (Methode C) |

### Beispiel 2:

### 4'-(5-Fluor-2,3-dihydroindol-1-carbonyl)-1'-methyl-4-(2-oxo-2,3-dihydroimidazo[4,5-b]-pyridin-1-yl)-3,4,5,6-tetrahydro-2H,1'H-[1,2']bipyridinyl-6'-on

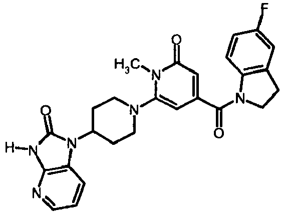

50 mg (0.11 mmol) 4'-(5-Fluor-2,3-dihydroindol-1-carbonyl)-4-(2-oxo-2,3-dihydroimidazo-[4,5-*b*]pyridin-1-yl)-3,4,5,6-tetrahydro-2*H*,1'*H*-[1,2']bipyridinyl-6'-on wurden in 0.50 mL DMSO bei RT vorgelegt. Dazu wurden 88 mg (0.27 mmol) Cäsiumcarbonat gegeben und 15 min gerührt. Dann wurde eine Lösung aus 6.5 µL (0.11 mmol) Methyliodid in DMSO zugegeben und 2h bei RT gerührt. Es wurde nochmals Methyliodid zugegeben (3.3 µL, 0.055 mmol) und weitere 2 h bei RT gerührt. Das Reaktionsgemisch wurde mittels präparativer HPLC aufgereinigt. Die Produkt enthaltenden Fraktionen wurden vereinigt und lyophilisiert.

| | |
|---|---|
| Ausbeute: | 15 mg (29% d. Th.) |
| ESI-MS: | m/z = 489 (M+H)⁺ |
| Rₜ(HPLC): | 1.88 min (Methode O) |

### Beispiel 3:

### 3-[4'-(5-Fluor-2,3-dihydroindol-1-carbonyl)-6'-oxo-3,4,5,6,1',6'-hexahydro-2H-1,2'-bi-pyridinyl-4-yl]-7-methoxy-1,3,4,5-tetrahydro-1,3-benzodiazepin-2-on

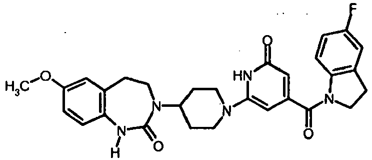

Diese Verbindung wurde analog zu 4'-(5-Fluor-2,3-dihydroindol-1-carbonyl)-4-(2-oxo-2,3-dihydroimidazo[4,5-*b*]pyridin-1-yl)-3,4,5,6-tetrahydro-2*H*,1'*H*-[1,2']bipyridinyl-6'-on aus 415 mg (0.761 mmol) 3-[4'-(5-Fluor-2,3-dihydroindol-1-carbonyl)-6'-methoxy-3,4,5,6-tetrahydro-2*H*-1,2'-bipyridinyl-4-yl]-7-methoxy-1,3,4,5-tetrahydro-1,3-benzodiazepin-2-on und 1.50 g (13.0 mmol) Pyridinhydrochlorid erhalten.

| | |
|---|---|
| Ausbeute: | 39 g (10% d. Th.) |
| ESI-MS: | m/z = 532 (M+H)⁺ |
| Rₜ(HPLC): | 4.80 min (Methode N) |

### Beispiel 4:

### 4'-(5-Fluor-3,3-dimethyl-2,3-dihydroindol-1-carbonyl)-4-(2-oxo-2,3-dihydroimidazo[4,5-b]-pyridin-1-yl)-3,4,5,6-tetrahydro-2H,1'H-[1,2']bipyridinyl-6'-on

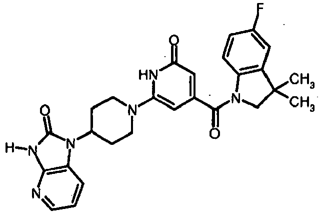

Diese Verbindung wurde analog zu 4'-(5-Fluor-2,3-dihydroindol-1-carbonyl)-4-(2-oxo-2,3-dihydroimidazo[4,5-*b*]pyridin-1-yl)-3,4,5,6-tetrahydro-2*H*,1'*H*-[1,2']bipyridinyl-6'-on aus 60 mg (0.12 mmol) 1-[4'-(5-Fluor-3,3-dimethyl-2,3-dihydroindol-1-carbonyl)-6'-methoxy-3,4,5,6-tetrahydro-2*H*-[1,2']bipyridinyl-4-yl]-1,3-dihydroimidazo[4,5-*b*]pyridin-2-on und 300 mg (2.6 mmol) Pyridinhydrochlorid erhalten.

| | |
|---|---|
| Ausbeute: | 20 mg (34% d. Th.) |
| ESI-MS: | m/z = 503 (M+H)⁺ |
| Rₜ(HPLC): | 1.17 min (Methode C) |

### Beispiel 5:

### 4'-(5-Fluor-3,3-dimethyl-2,3-dihydroindol-1-carbonyl)-1'-methyl-4-(2-oxo-2,3-dihydro-imidazo[4,5-b]pyridin-1-yl)-3,4,5,6-tetrahydro-2H,1'H-[1,2']bipyridinyl-6'-on

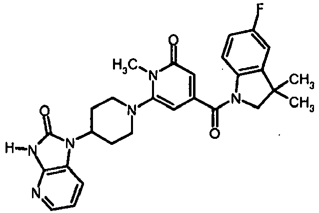

Diese Verbindung wurde analog zu 4'-(5-Fluor-2,3-dihydroindol-1-carbonyl)-1'-methyl-4-(2-oxo-2,3-dihydroimidazo[4,5-b]pyridin-1-yl)-3,4,5,6-tetrahydro-2*H*,1'*H*-[1,2']bipyridinyl-6'-on aus 20 mg (0.040 mmol) 4'-(5-Fluor-3,3-dimethyl-2,3-dihydroindol-1-carbonyl)-4-(2-oxo-2,3-dihydroimidazo[4,5-*b*]pyridin-1-yl)-3,4,5,6-tetrahydro-2*H*,1'*H*-[1,2']bipyridinyl-6'-on, 3.8 µL (0.060 mmol) Methyliodid und 33 mg (0.10 mmol) Cäsiumcarbonat in 0.50 mL DMSO erhalten.

| | |
|---|---|
| Ausbeute: | 15 mg (73% d. Th.) |
| ESI-MS: | m/z = 517 (M+H)⁺ |
| Rₜ(HPLC): | 2.16 min (Methode C) |

### Beispiel 6:

### 4'-(4,5-Difluor-2,3-dihydro-indol-1-carbonyl)-4-(2-oxo-2,3-dihydro-imidazo[4,5-b]pyridin-1-yl)-3,4,5,6-tetrahydro-2H,1'H-[1,2']bipyridinyl-6'-on

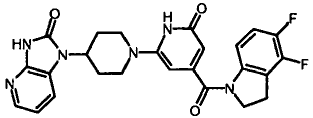

Ein gut gerührtes Gemisch aus 100 mg (0.197 mmol) 1-[4'-(4,5-Difluor-2,3-dihydro-indol-1-carbonyl)-6'-methoxy-3,4,5,6-tetrahydro-2H-[1,2']bipyridinyl-4-yl]-1,3-dihydro-imidazo-[4,5-b]pyridin-2-on und 0.500 g (4.33 mmol) Pyridinhydrochlorid wurde 7 min mit dem Heißluftfön in der Schmelze gehalten. Nach dem Abkühlen der Reaktion wurde der Ansatz in DMF gelöst und mittels präparativer HPLC aufgereinigt. Die Produkt enthaltenden Fraktionen wurden vereinigt und lyophilisiert.

| | |
|---|---|
| Ausbeute: | 56 mg (58% d. Th.) |
| ESI-MS: | m/z = 493 (M+H)⁺ |
| Rₜ(HPLC): | 1.27 min (Methode C) |

### Beispiel 7:

### 6'-(5-Fluor-2,3-dihydro-indol-1-carbonyl)-4-(2-oxo-2,3-dihydro-imidazo[4,5-b]pyridin-1-yl)-3,4,5,6-tetrahydro-2H,1'H-[1,4']bipyridinyl-2'-on

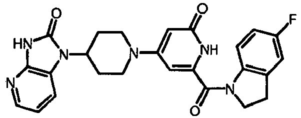

Ein gut gerührtes Gemisch aus 40 mg (0.082 mmol) 1-[6'-(5-Fluor-2,3-dihydro-indol-1-carbonyl)-2'-methoxy-3,4,5,6-tetrahydro-2H-[1,4']bipyridinyl-4-yl]-1,3-dihydro-imidazo-[4,5-b]pyridin-2-on und 0.020 g (1.7 mmol) Pyridinhydrochlorid wurde 2 min mit dem Heißluftfön in der Schmelze gehalten. Nach dem Abkühlen der Reaktion wurde der Ansatz in DMF gelöst und mittels präparativer HPLC aufgereinigt. Die Produkt enthaltenden Fraktionen wurden vereinigt und lyophilisiert.

| | |
|---|---|
| Ausbeute: | 22 mg (57% d. Th.) |
| ESI-MS: | m/z = 475 (M+H)⁺ |
| Rₜ(HPLC): | 1.1 min (Methode C) |

Die nachfolgenden Beispiele beschreiben die Herstellung pharmazeutischer Anwendungsformen, die als Wirkstoff eine beliebige Verbindung der allgemeinen Formeln **Ia** und **Ib** enthalten:

### Beispiel I

### Kapseln zur Pulverinhalation mit 1 mg Wirkstoff

### Zusammensetzung:

### 1 Kapsel zur Pulverinhalation enthält:

| | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

### Inhalationslösung für Respimat^{®} mit 1 mg Wirkstoff

### Zusammensetzung:

### 1 Hub enthält:

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

### Inhalationslösung für Vernebler mit 1 mg Wirkstoff

### Zusammensetzung:

### 1 Fläschchen enthält:

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

### Wirkstoff, Natriumchlorid und Benzalkoniumchlorid werden in Wasser gelöst.

### Beispiel IV

### Treibgas-Dosieraerosol mit 1 mg Wirkstoff

### Zusammensetzung:

### 1 Hub enthält:

| | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

### Nasalspray mit 1 mg Wirkstoff

### Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

### Injektionslösung mit 5 mg Wirksubstanz pro 5 ml

### Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

### Injektionslösung mit 100 mg Wirksubstanz pro 20 ml

### Zusammensetzung:

| | |
|---|---|
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

### Lyophilisat mit 10 mg Wirksubstanz

### Zusammensetzung:

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

### Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

### Tabletten mit 20 mg Wirksubstanz

### Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässrigen Lösung von Povidon granulieren; mit Magnesiumstearat mischen; auf einer Tablettenpresse abpressen; Tablettengewicht 200 mg.

### Beispiel X

### Kapseln mit 20 mg Wirksubstanz

### Zusammensetzung:

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

### Zäpfchen mit 50 mg Wirksubstanz

### Zusammensetzung:

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

### Injektionslösung mit 10 mg Wirksubstanz pro 1 mL

### Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Verbindungen der allgemeinen Formeln **Ia** und **Ib** 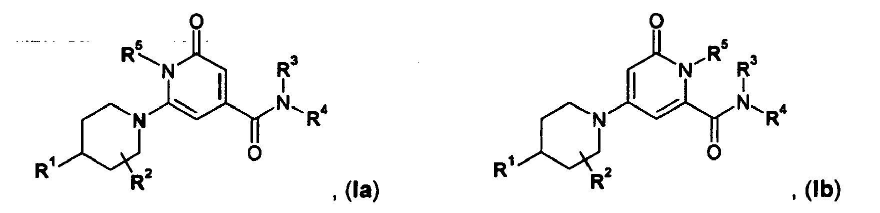 in der
**R¹** eine Gruppe ausgewählt aus 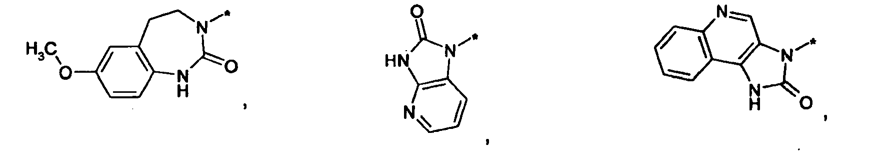 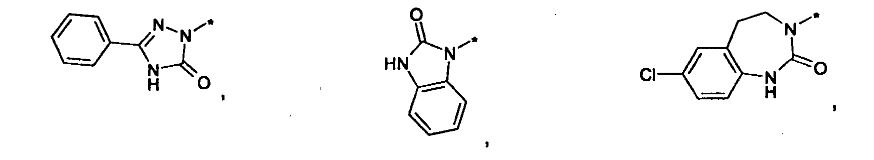 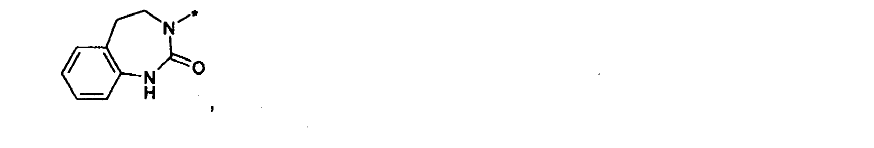
**R²** H,
**R³**
(a) H,
(b) C₁₋₃-Alkyl,
(c) eine C₁₋₃-Alkylgruppe, in der jede Methylengruppe mit bis zu zwei Fluoratomen und jede Methylgruppe mit bis zu drei Fluoratomen substituiert ist, und
**R⁴** H oder eine Gruppe ausgewählt aus 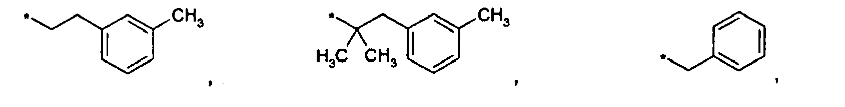 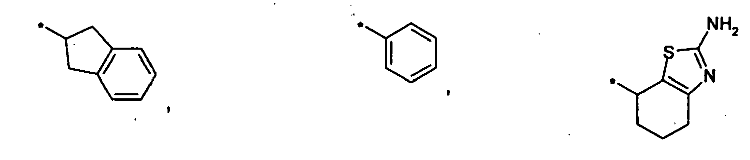 oder
**R³** und **R⁴** zusammen mit dem Stickstoffatom, an das sie gebunden sind, eine Gruppe ausgewählt aus 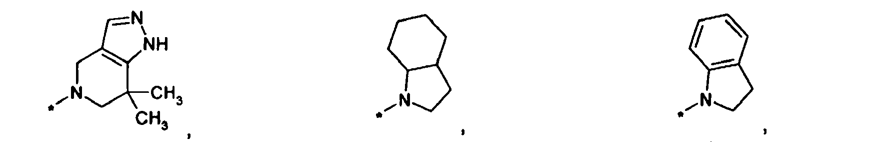 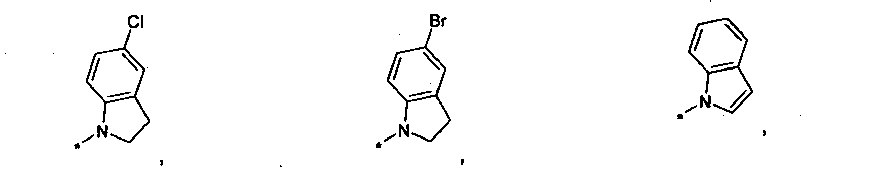 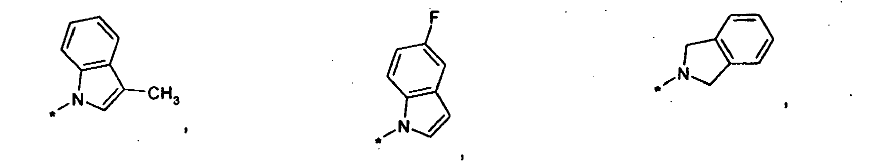 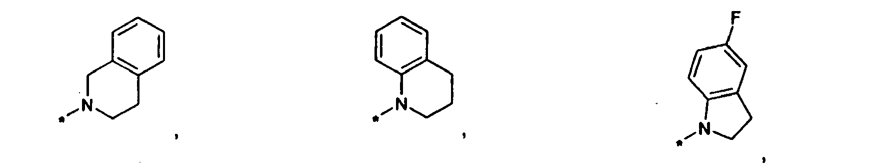 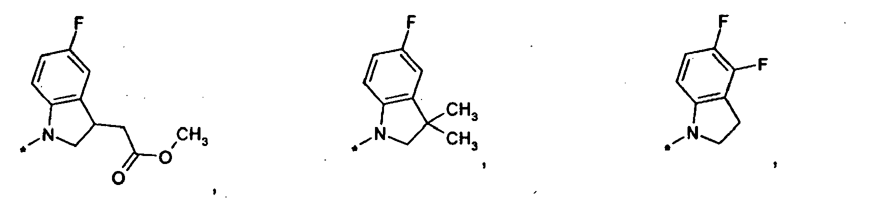 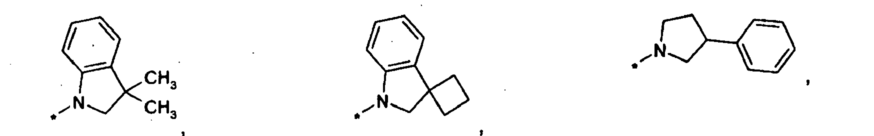 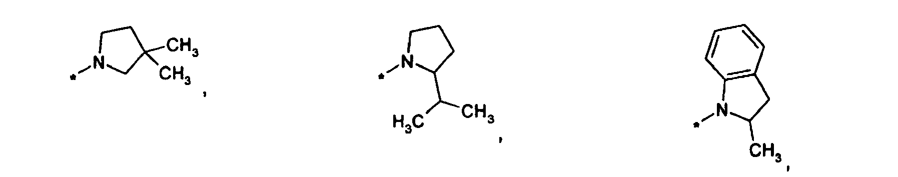 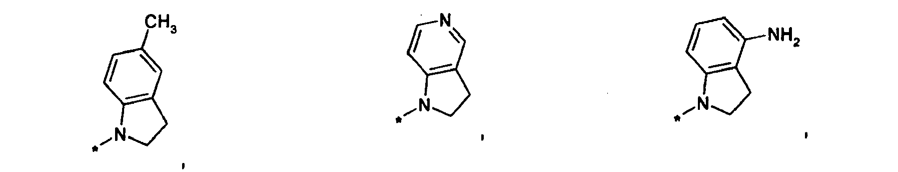 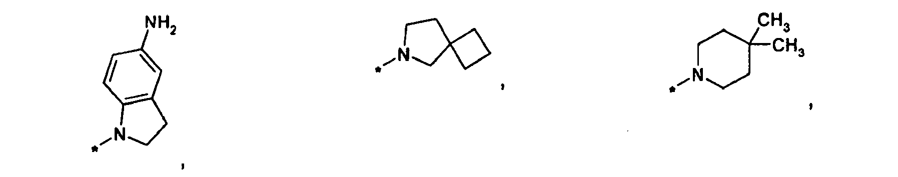 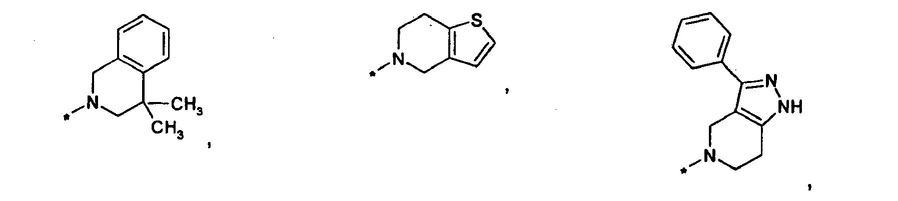 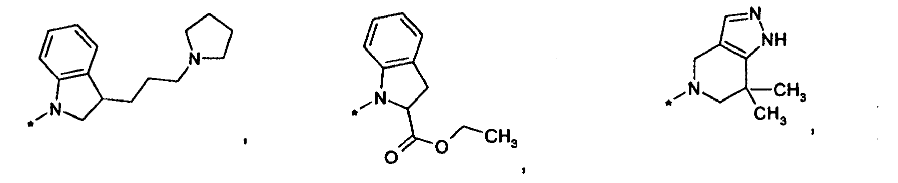 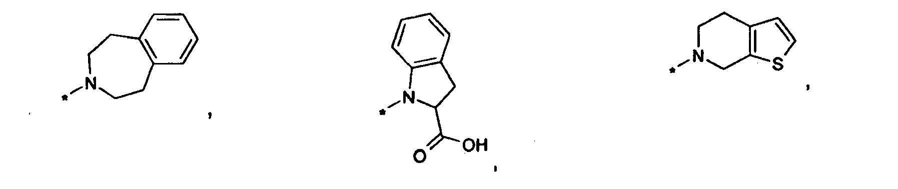 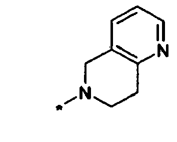 und
**R⁵** H oder C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. Folgende Verbindungen der allgemeinen Formeln **Ia** und **Ib** gemäß Anspruch 1:
| **Nr.** | **Struktur** |
|---|---|
| (1) | 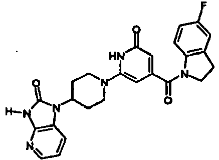 |
| (2) | 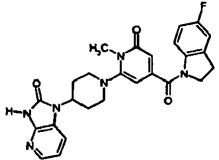 |
| (3) | 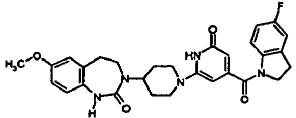 |
| (4) | 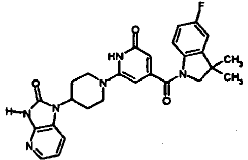 |
| (5) | 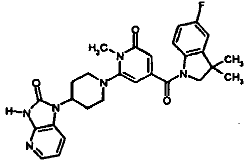 |
| (6) | 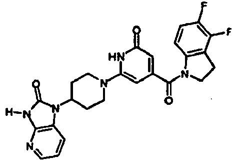 |
| (7) | 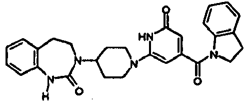 |
| (8) | 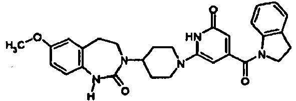 |
| (9) | 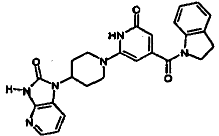 |
| (10) | 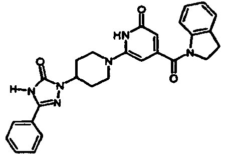 |
| (11) | 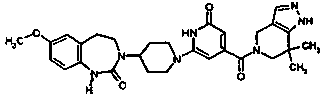 |
| (12) | 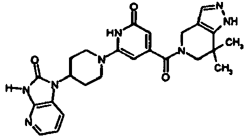 |
| (13) | 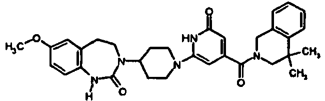 |
| (14) | 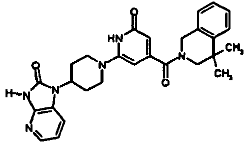 |
| (15) | 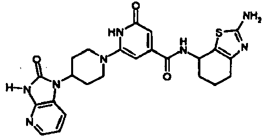 |
| (16) | 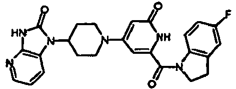 |
deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

3. Physiologisch verträgliche Salze der Verbindungen nach einem der Ansprüche 1 bis 2 mit anorganischen oder organischen Säuren oder Basen.

4. Arzneimittel, enthaltend eine Verbindung nach einem der Ansprüche 1 bis 2 oder ein physiologisch verträgliches Salz gemäß Anspruch 3 neben gegebenenfalls einem oder mehreren inerten Trägerstoffen und/oder Verdünnungsmitteln.

5. Verwendung einer Verbindung nach einem der Ansprüche 1 bis 3 zur Herstellung eines Arzneimittels zur akuten und prophylaktischen Behandlung von Kopfschmerzen, insbesondere Migräne- und Cluster-Kopfschmerz sowie Spannungskopfschmerzen.

6. Verwendung einer Verbindung nach einem der Ansprüche 1 bis 3 zur Herstellung eines Arzneimittels zur Behandlung von nicht-insulinabhängigem Diabetes mellitus ("NIDDM"), cardiovaskulärer Erkrankungen, Morphintoleranz, Clostridiumtoxin-bedingter Durchfallerkrankungen, Erkrankungen der Haut, insbesondere thermischer und strahlenbedingter Hautschäden inklusive Sonnenbrand, Lichen, Prurigo, pruriginöse Toxidermien sowie schwerer Juckreiz, entzündlicher Erkrankungen, z.B. entzündlicher Gelenkerkrankungen (Osteoarthritis, rheumatoide Arthritis, neurogene Arthritis), generalisierter Weichteilrheumatismus (Fibromyalgie), neurogener Entzündungen der oralen Mucosa, entzündlicher Lungenerkrankungen, allergischer Rhinitis, Asthma, COPD, Erkrankungen, die mit einer überschießenden Gefäßerweiterung und dadurch bedingter verringerter Gewebedurchblutung einhergehen, z.B. Schock und Sepsis, chronischer Schmerzerkrankungen, wie z.B. diabetische Neuropathien, durch Chemotherapien induzierter Neuropathien, HIV-induzierter Neuropathien, postherpetischer Neuropathien durch Gewebetrauma induzierter Neuropathien, trigeminaler Neuralgien, temporomandibulärer Dysfunktionen, CRPS (complex regional pain syndrome), Rückenschmerzen, und viszeraler Erkrankungen, wie z.B. irritable bowel syndrome (IBS), inflammatory bowel syndrome, zur lindernden Wirkung auf Schmerzzustände im allgemeinen oder zur präventiven oder akut-therapeutischen Behandlung der Symptomatik menopausaler, durch Gefäßerweiterung und erhöhten Blutfluss verursachter Hitzewallungen östrogendefizienter Frauen sowie hormonbehandelter Prostatakarzinompatienten und Kastraten.

7. Verwendung einer Verbindung nach einem der Ansprüche 1 bis 3 zur Herstellung eines Arzneimittels zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

8. Verfahren zur Herstellung eines Arzneimittels gemäß Anspruch 4, **dadurch gekennzeichnet, dass** auf nichtchemischem Weg eine Verbindung nach einem der Ansprüche 1 bis 3 in einen oder mehrere inerte Trägerstoffe und/oder Verdünnungsmittel eingearbeitet wird.

## Claims

1. Compounds of general formulae **Ia** and **Ib** 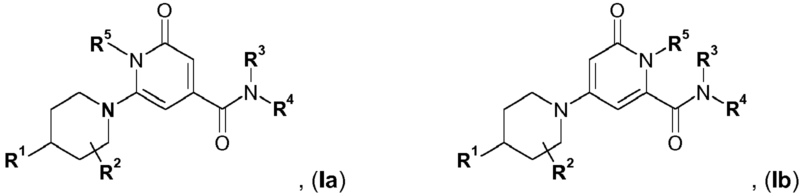 wherein
**R¹** denotes a group selected from 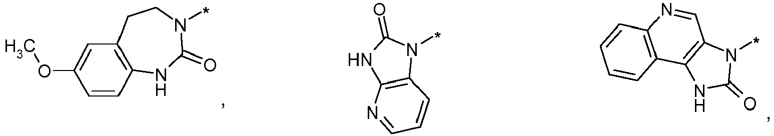 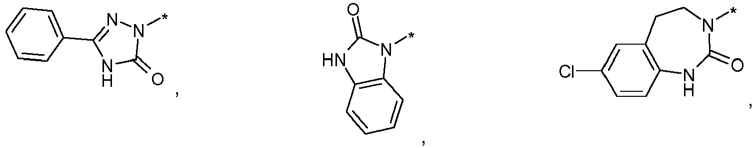 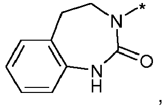
**R²** denotes H,
**R³** denotes
(a) H,
(b) C₁₋₃-alkyl,
(c) a C₁₋₃-alkyl group wherein each methylene group is substituted by up to two fluorine atoms and each methyl group is substituted by up to three fluorine atoms, and
**R⁴** denotes H or a group selected from 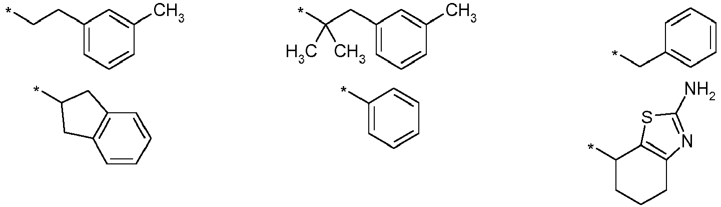 or
**R³** and **R⁴** together with the nitrogen atom to which they are bound denote a group selected from 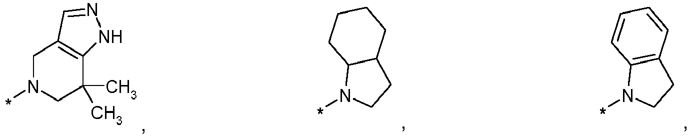 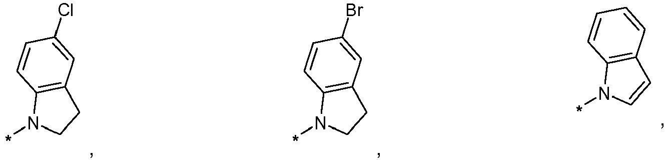 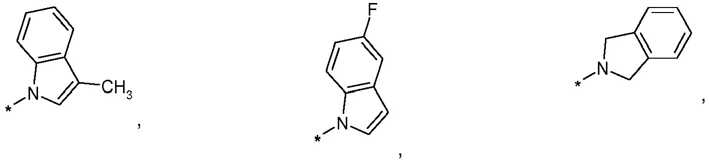 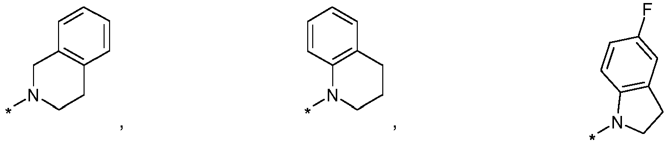 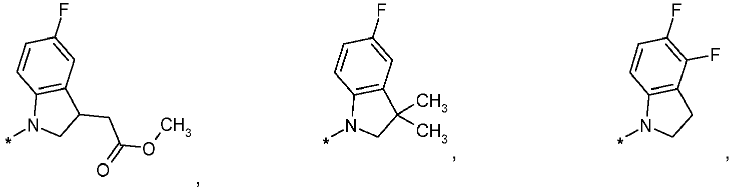 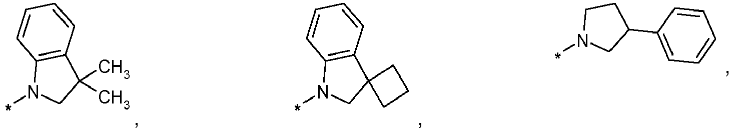 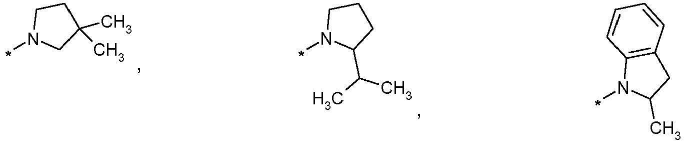 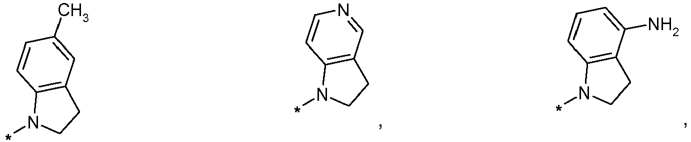 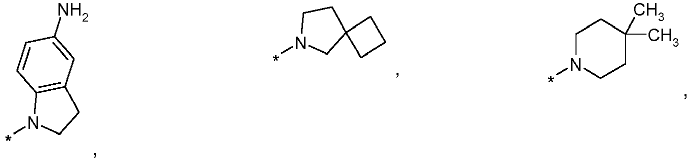 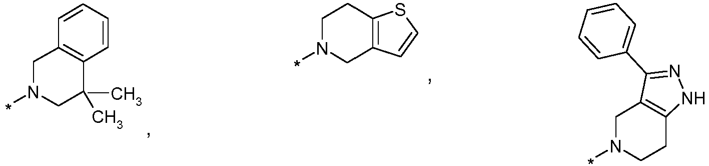 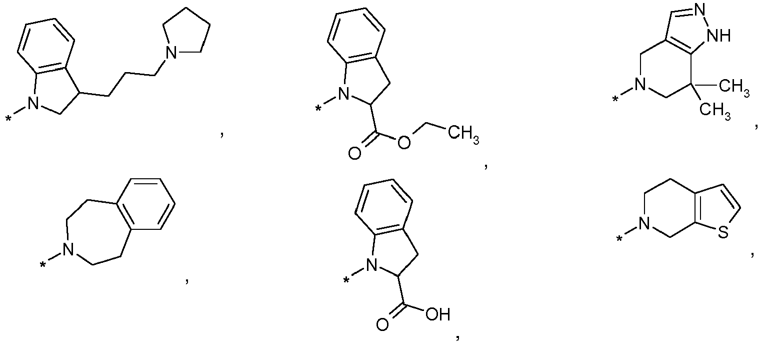 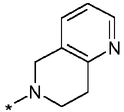 and
**R⁵** denotes H or C₁₋₃-alkyl,
the tautomers, the diastereomers, the enantiomers, the hydrates, the mixtures thereof and the salts thereof as well as the hydrates of the salts, particularly the physiologically acceptable salts thereof with inorganic or organic acids or bases.

2. The following compounds of general formulae **Ia** and **Ib** according to claim 1:
| **No.** | **Structure** |
|---|---|
| (1) | 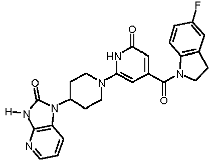 |
| (2) | 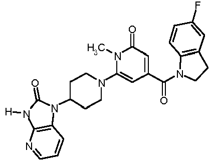 |
| (3) | 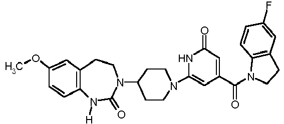 |
| (4) | 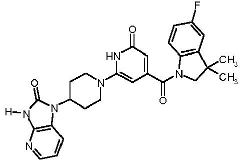 |
| (5) | 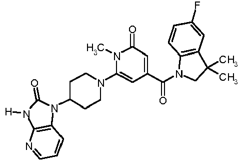 |
| (6) | 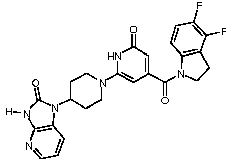 |
| (7) | 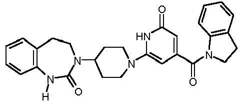 |
| (8) | 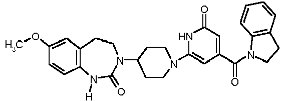 |
| (9) | 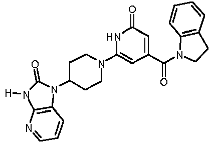 |
| (10) | 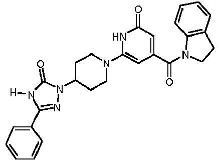 |
| (11) | 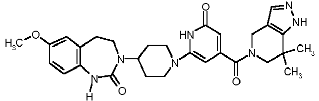 |
| (12) | 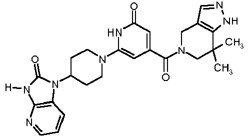 |
| (13) | 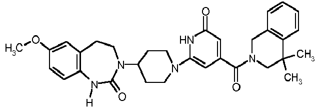 |
| (14) | 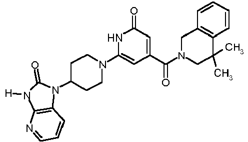 |
| (15) | 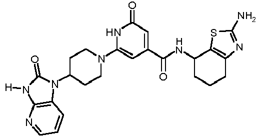 |
| (16) | 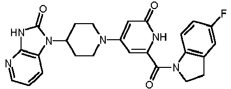 |
the enantiomers, the diastereomers, the hydrates, the mixtures thereof and the salts thereof as well as the hydrates of the salts, particularly the physiologically acceptable salts thereof with inorganic or organic acids or bases.

3. Physiologically acceptable salts of the compounds according to one of claims 1 to 2 with inorganic or organic acids or bases.

4. Medicaments, containing a compound according to one of claims 1 to 2 or a physiologically acceptable salt according to claim 3 optionally together with one or more inert carriers and/or diluents.

5. Use of a compound according to one of claims 1 to 3 for preparing a medicament for the acute and prophylactic treatment of headaches, particularly migraine and cluster headaches and tension headaches.

6. Use of a compound according to one of claims 1 to 3 for preparing a medicament for the treatment of non-insulin-dependent diabetes mellitus ("NIDDM"), cardiovascular diseases, morphine tolerance, diarrhoea caused by clostridium toxin, skin diseases, particularly thermal and radiation-induced skin damage including sunburn, lichen, pruritis, pruritic toxidermies and severe itching, inflammatory diseases, e.g. inflammatory diseases of the joints (osteoarthritis, rheumatoid arthritis, neurogenic arthritis), generalised soft-tissue rheumatism (fibromyalgia), neurogenic inflammation of the oral mucosa, inflammatory lung diseases, allergic rhinitis, asthma, COPD, diseases accompanied by excessive vasodilatation and resultant reduced blood supply to the tissues, e.g. shock and sepsis, chronic pain, e.g. diabetic neuropathies, neuropathies induced by chemotherapy, HIV-induced neuropathies, postherpetic neuropathies, neuropathies induced by tissue trauma, trigeminal neuralgias, temporomandibular dysfunctions, CRPS (complex regional pain syndrome), back pain, and visceral complaints, such as e.g. irritable bowel syndrome (IBS), inflammatory bowel syndrome, for relieving pain in general, or for preventive or acute therapeutic treatment of the symptoms of menopausal hot flushes caused by vasodilatation and increased blood flow in oestrogen-deficient women and hormone-treated patients with prostate carcinoma and castrated men.

7. Use of a compound according to one of claims 1 to 3 for preparing a medicament for the acute and prophylactic treatment of migraine and cluster headaches, for the treatment of irritable bowel syndrome (IBS) and for the preventive and acute therapeutic treatment of hot flushes in oestrogen-deficient women.

8. Method of preparing a medicament according to claim 4, **characterised in that** a compound according to one of claims 1 to 3 is incorporated in one or more inert carriers and/or diluents by a non-chemical method.

## Revendications

1. Composés de formules générales Ia et Ib 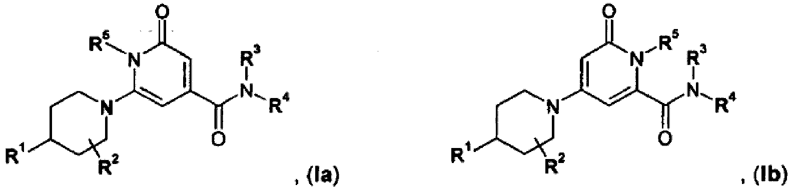 ou
R¹ représente un groupe choisi parmi 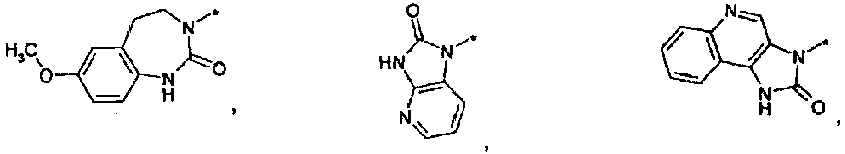 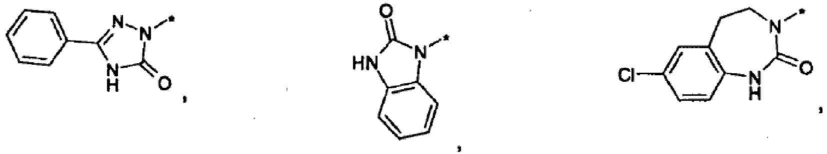 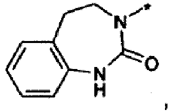
R² représente H,
R³ représente
(a) H,
(b) alkyle en C₁₋₃,
(c) un groupe alkyle en C₁₋₃, dans lequel chaque groupe méthylène est substitué par jusqu'à deux atomes de fluore et chaque groupe méthyle est substitué par jusqu'à trois atomes de fluor, et
R⁴ représente H ou un groupe choisi parmi 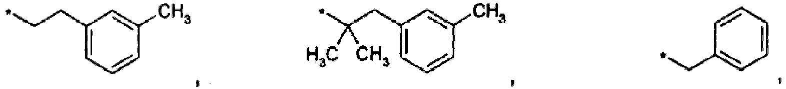 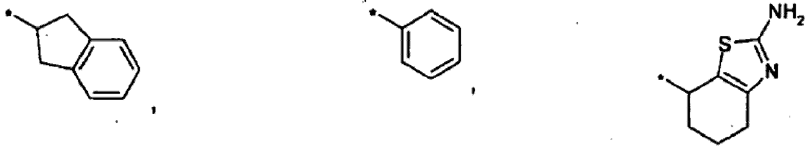 ou
R³ et R⁴ conjointement avec l'atome d'azote, auquel ilssont liés, représentent un groupe choisi parmi 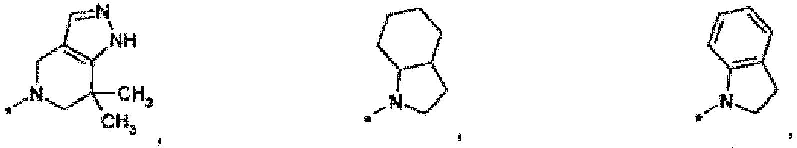 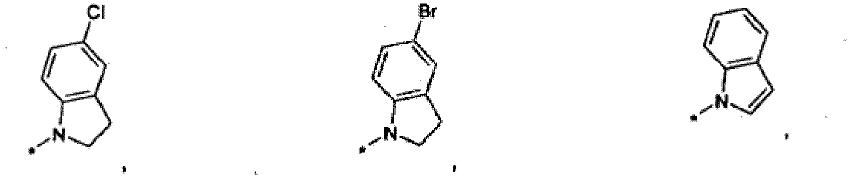 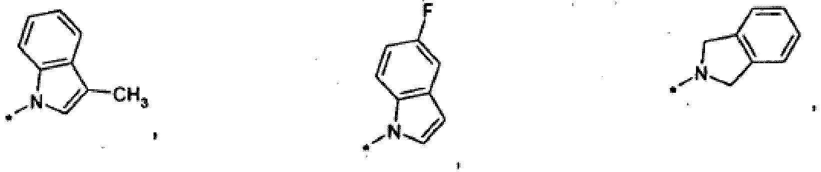 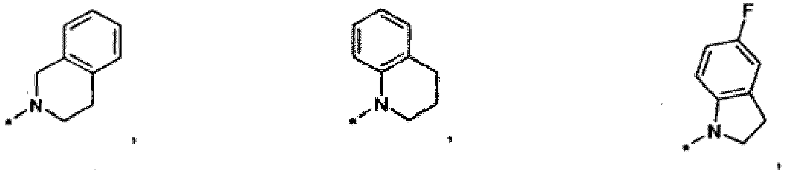 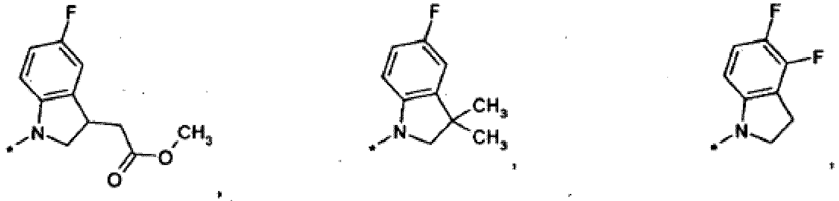 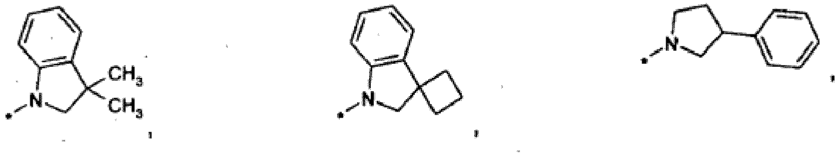 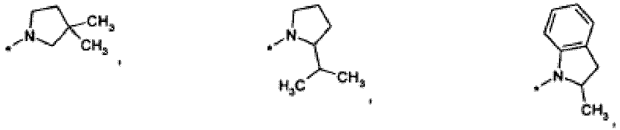 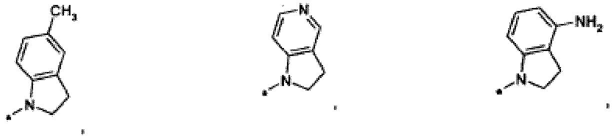 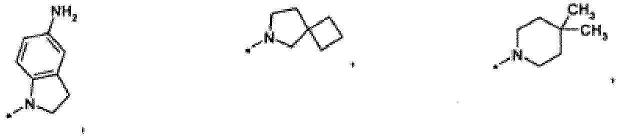 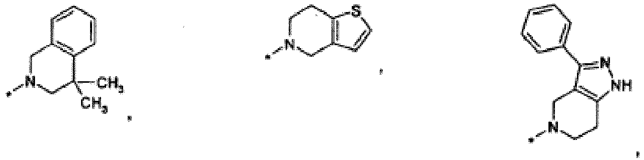 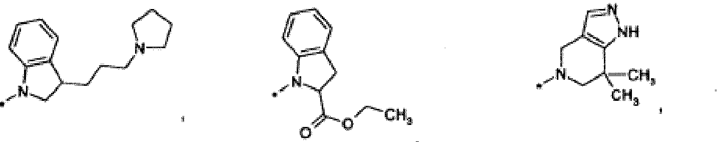 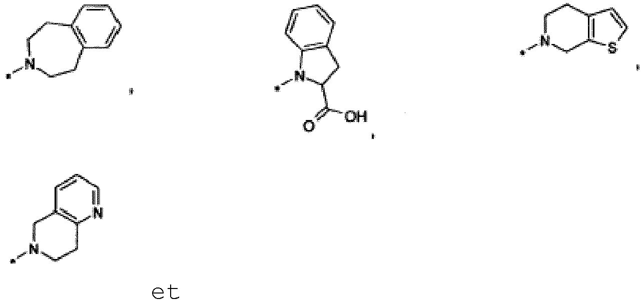
R⁵ représente H ou alkyle en C₁₋₃,
leurs tautomères, leurs diastéréoisomères, leurs énantiomères, leurs hydrates, leurs mélanges et leurs sels ainsi que les hydrates des sels, en particulier leurs sels physiologiquement acceptables avec des acides ou des bases inorganiques ou organiques.

2. Composés suivants de formules générales Ia et Ib selon la revendication 1 :
| N° | Structure |
|---|---|
| (1) | 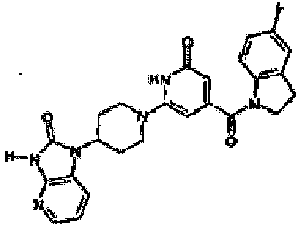 |
| (2) | 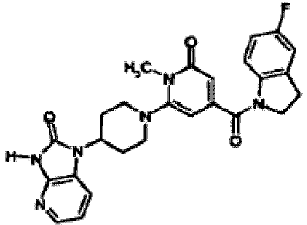 |
| (3) | 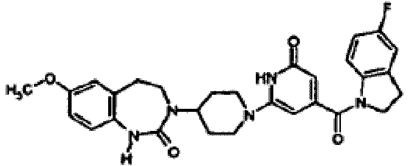 |
| (4) | 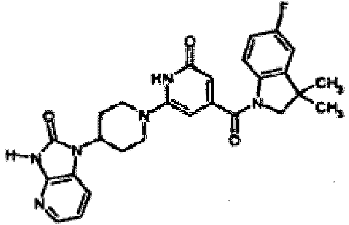 |
| (5) | 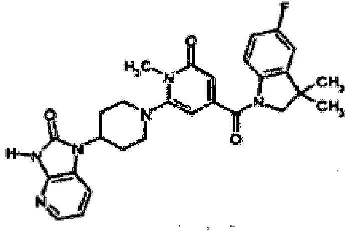 |
| (6) | 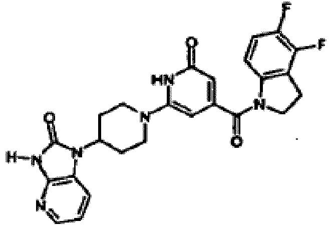 |
| (7) | 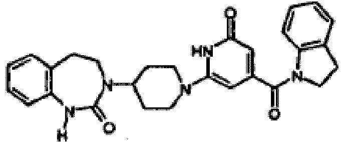 |
| (8) | 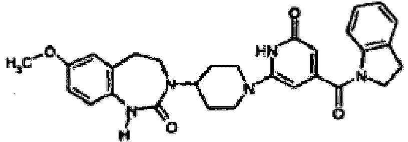 |
| (9) | 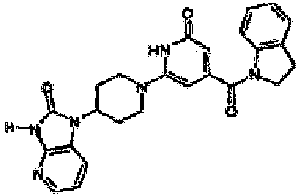 |
| (10) | 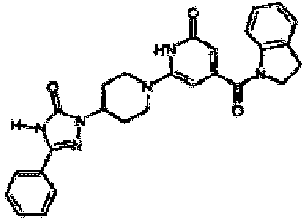 |
| (11) | 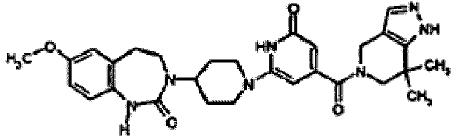 |
| (12) | 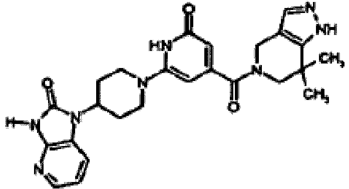 |
| (13) | 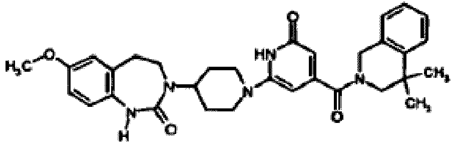 |
| (14) | 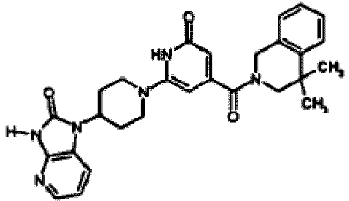 |
| (15) | 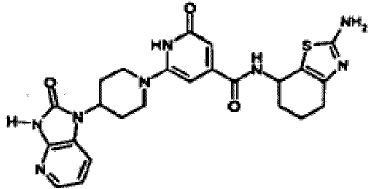 |
| (16) | 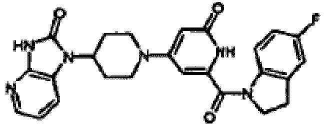 |
leurs énantiomères, leurs diastéréoisomères, leurs hydrates, leurs mélanges et leurs sels ainsi que les hydrates des sels, en particulier leurs sels physiologiquement acceptables avec des acides ou des bases inorganiques ou organiques.

3. Sels physiologiquement acceptables des composés selon l'une quelconque des revendications 1 à 2 avec des acides ou des bases inorganiques ou organiques.

4. Médicament, contenant un composé selon l'une quelconque des revendications 1 à 2 ou contenant un sel physiologiquement acceptable selon la revendication 3 en plus, le cas échéant, d'un ou de plusieurs excipients et/ou agents de dilution inertes.

5. Utilisation d'un composé selon l'une quelconque des revendications 1 à 3 pour la fabrication d'un médicament pour le traitement aigu et prophylactique de céphalées, en particulier de migraines ou d'algies vasculaires de la face ainsi que de céphalées de tension.

6. Utilisation d'un composé selon l'une quelconque des revendications 1 à 3 pour la fabrication d'un médicament pour le traitement du diabète non insulino-dépendant (DNID), de maladies cardiovasculaires, de la tolérance à la morphine, de diarrhées liées aux toxines produites par le clostridium, de maladies de la peau, en particulier lésions cutanées thermiques et liées aux rayons, y compris coups de soleil, lichen, prurigo, toxidermies prurigineuses ainsi que des démangeaisons sévères, de maladies inflammatoires, par exemple maladies articulaires inflammatoires (ostéoarthrite, poly-arthrite rhumatoïde, arthrite neurogène), rhumatisme des tissus mous généralisé (fibromyalgie), inflammations neurogènes de la muqueuse buccale, maladies pulmonaires inflammatoires, rhinite allergique, asthme, BPCO, de maladies, qui vont de pair avec une vasodilatation excessive et avec une irrigation sanguine réduite associée, par exemple choc et sepsis, de douleurs chroniques, par exemple neuropathies diabétiques, neuropathies induites par des chimiothérapies, neuropathies induites par VIH, neuropathies post-herpétiques, neuropathies induites par des traumatismes tissulaires, névralgies trigéminales, troubles temporomandibulaires, syndrome douloureux régional complexe (SDRC), douleurs au dos, et de maladies viscérales, par exemple syndrome du côlon irritable (SCI), syndrome inflammatoire de l'intestin, pour un effet apaisant des états douloureux en général ou pour le traitement préventif ou thérapeutique aigu des symptômes de bouffées de chaleur liées à la ménopause, provoquées par la vasodilatation et l'augmentation du flux sanguin, de femmes déficientes en oestrogène, ainsi que de patients atteints du cancer de la prostate sous traitement hormonal et de castrats.

7. Utilisation d'un composé selon l'une quelconque des revendications 1 à 3 pour la fabrication d'un médicament pour le traitement aigu et prophylactique de migraines et d'algies vasculaires de la face, pour le traitement du syndrome du côlon irritable (SCI) et pour le traitement préventif et thérapeutique aigu de bouffées de chaleur de femmes déficientes en estrogène.

8. Procédé de fabrication d'un médicament selon la revendication 4, **caractérisé en ce qu'**un composé selon l'une quelconque des revendications 1 à 3 est incorporé dans un ou plusieurs excipients et/ou agents de dilution inertes par voie non chimique.
